# EUROPEAN PATENT SPECIFICATION

(11) **EP 3 819 636 B1**
(45) Date of publication and mention of the grant of the patent: **11.10.2023**
(21) Application number: 19207864.0
(22) Date of filing: 08.11.2019
(51) Int. Cl.: G01N 33/46, B32B 21/14

(54) **AN ARRANGEMENT COMPRISING WOOD BASED ARTICLES AND A MOISTURE SENSOR, AND A METHOD FOR MANUFACTURING THE SAME**
ANORDNUNG MIT AUF HOLZBASIERTEN ARTIKELN UND FEUCHTIGKEITSSENSOR SOWIE VERFAHREN ZUR HERSTELLUNG DAVON
AGENCEMENT COMPRENANT DES ARTICLES À BASE DE BOIS ET UN CAPTEUR D'HUMIDITÉ ET SON PROCÉDÉ DE FABRICATION

(43) Date of publication of application: 12.05.2021
(73) Proprietor: UPM Plywood Oy, 15140 Lahti (FI)
(72) Inventor: HÄRKÖNEN, Riku, 15240 LAHTI (FI)
(74) Representative: Berggren Oy

(56) References cited:
- EP-A1- 1 345 027
- RU-C1- 2 039 973
- US-A- 5 621 391
- US-A1- 2014 009 174

## Description

### Technical field

The invention relates to shipments comprising wood based articles. The invention relates to shipments comprising wood based articles having a determined moisture content. The invention relates to remotely readable shipments. The invention relates to wood veneers. The invention relates to methods for manufacturing wood floor. The invention relates to forming shipments for manufacturing wood floor.

### Background

Wood is a material that absorbs and emits moisture depending on the relative humidity of the environment and the moisture content of the wood. Moreover, wood swells when it absorbs moisture and contracts when it releases moisture. For these reasons, in some fields it is important that wood, when used as raw material, has a proper moisture content. As an example, when manufacturing wood boards for floorings, the moisture content is important, as known e.g. from US6695944.

Methods for determining moisture content of wood material include measurements by drying. Correspondingly, a sample may be dried to a moisture content of substantially zero, and the amount of evaporated moisture can be determined, e.g. from the mass decrease during drying. The wood material itself may be tested, or in the alternative, a wood material dummy sample (e.g. a piece of wood, possibly other wood than the rest of the shipment) may be taken from the same shipment, and, provided that there is a moisture equilibrium in the shipment, the moisture content of the dummy sample can be determined instead of testing the other wood materials, possibly more expensive materials, of the shipment. Thus, by doing such measurements, a wood board manufacturer can be sure that the moisture content of the raw material, which is also wood based material, is within a proper range. However, the process is time consuming, since drying of the samples may take a lot of time.

Also other methods for such measurements are known. In this field, US 5,621,391 discloses a probe that is insertable in a gap between layers of wooden beams. The probe is connected by a wire to a hand-held moisture content meter. Also EP 1 345 027 discloses a probe that is insertable in a gap between layers of lumber. Moisture content is measurable from an electric resistance. In addition, RU 2 039 973 discloses two electrodes can be inserted in a gap between layers of lumber. Moisture content is measurable us radio-technical measurement principle. Finally, US 2014/0009174 a wood monitoring system for measuring lumber moisture. The system comprises wireless sensors.

### Summary

It has been found that a moisture content of a shipment can be more easily detected by a remotely readable moisture content sensing device, which is arranged within the shipment. However, it has been found that measurements are more reliable from certain locations of the shipment than from other locations. Within the context of the present invention, the shipment is an arrangement. The arrangement comprises articles of wood based material, having sheet-like shape, and arranged on top of each other. It has been found that a remotely readable sensor device configured to measure a value indicative of a moisture content functions well, when at least a part of the remotely readable sensor device is arranged in between a first article and a second article. Thus, the moisture content of the arrangement can be easily measured with a reader device. Such an arrangement is more specifically disclosed in claim 1.

Moreover, a method for forming such an arrangement is presented. Such a method is more specifically disclosed in claim 12. In a preferable embodiment, the remotely readable sensor device configured to measure a value indicative of a moisture content is arranged on a relatively thin carrier, which comprises a holder for a tool for inserting the carrier. By using the tool, the carrier can be pushed in between the articles from the holder, whereby also the remotely readable moisture content sensing device will be arranged in between two articled. The corresponding method and arrangement are disclosed more specifically in claims 13, 14, 3, and 4, respectively.

### Brief description of the drawings

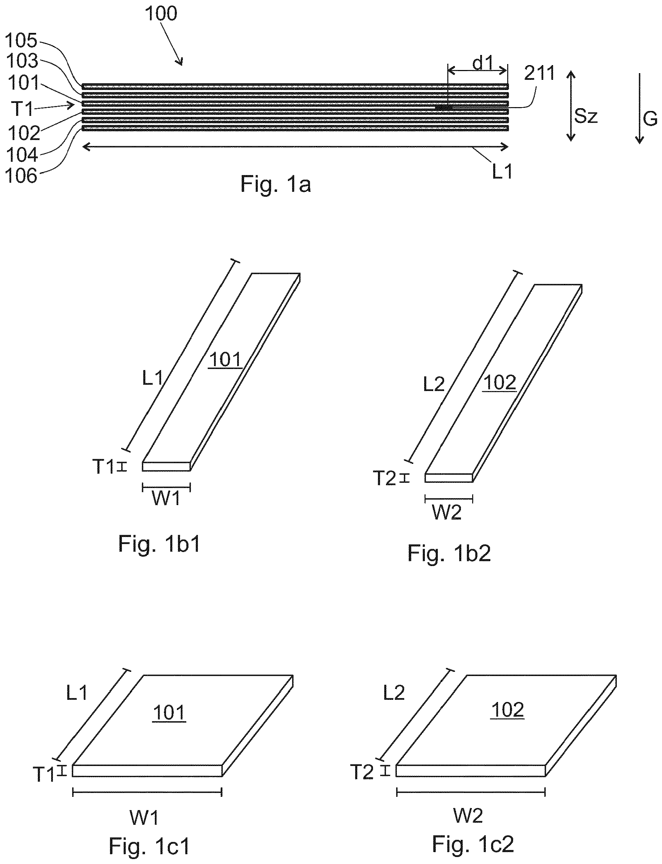

- Fig. 1a: shows, in a side view, an arrangement comprising articles and a remotely readable sensor device,

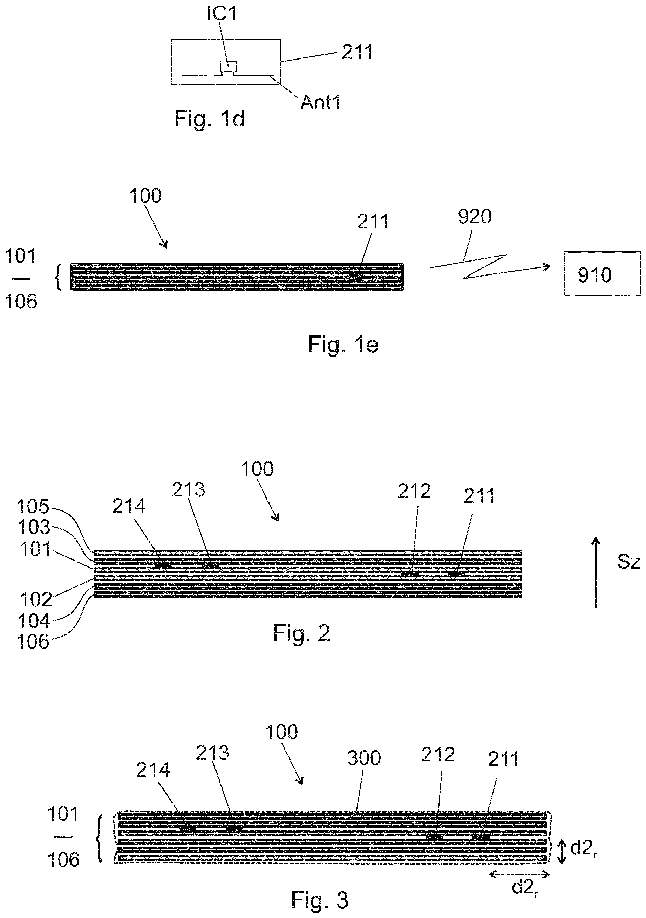

- Fig. 1 b1: shows, in a perspective view, a narrow first article,
- Fig. 1b2: shows, in a perspective view, a narrow second article,
- Fig. 1c1: shows, in a perspective view, a wide first article,
- Fig. 1c2: shows, in a perspective view, a wide second article,
- Fig. 1d: shows schematically a remotely readable sensor device,
- Fig. 1e: shows schematically a reader device receiving information from the remotely readable sensor device,
- Fig. 2: shows, in a side view, an arrangement comprising articles and remotely readable sensor devices,
- Fig. 3: shows, in a side view, an arrangement comprising wrapping,
- Fig. 4: shows, in a side view, an arrangement comprising wrapping provided with an information carrier,
- Figs. 5a and 5b: show a first article consisting of a wooden veneer and a second article consisting of a wooden veneer,
- Fig. 6a: shows, in a top view, a carrier that is provided with the remotely readable sensor device,
- Fig. 6b: shows, in a side view, an arrangement comprising the carrier,
- Fig. 7a: shows, in a top view, an embodiment of a carrier,
- Fig. 7b: shows, in a perspective view, the carrier of Fig. 7a,
- Fig. 7c: shows, in a top view, an embodiment of a carrier,
- Fig. 7d: shows, in a side view, a detailed view of a holder of a carrier,
- Fig. 7e: shows, in a side view, a detailed view of a holder of a carrier,
- Fig. 8: shows, in a perspective view, an arrangement wherein the articles are veneers and the sensor device is arranged on a carrier,
- Figs. 9a to 9d: show a method for forming an arrangement,
- Figs. 10a to 10c: show a method for removing a carrier from the arrangement,
- Fig. 11a: shows an embodiment of an arrangement having two carriers,
- Fig. 11b: shows an embodiment of an arrangement having two carriers and wrapping,
- Fig. 12a: shows an embodiment of an arrangement having multiple articles on top of each other and side by side, and
- Fig. 12b: shows an embodiment of an arrangement having multiple articles on top of each other only, and
- Fig. 13: shows some measures of a carrier.

In the figures, Sz, Sx, and Sy denote three mutually orthogonal directions. In a typical shipment, Sz is vertical. G denotes the direction of gravity.

### Detailed description

Fig. 1a shows, in a side view an arrangement 100. The arrangement 100 may be a shipment of articles 101, 102, 103, 104, 105, and 106. The arrangement 100 comprises a first article 101 and a second article 102. Within this description the term "article" refers to a sheet like object made from wood based material. The term "sheet like" refers to an object, which has a thickness, a width that is substantially greater than the thickness and a length that is also substantially greater than the thickness. Therefore, according to the present invention, the first article 101 is made of wood based material. Referring to Figs. 1b1 and 1c1, the first article has a first length L1, a first width W1, and a first thickness T1. Since the thickness is substantially smaller than the length, a ratio L1/T1 of the first length L1 to the first thickness T1 is at least 10 (ten). Since the thickness is substantially smaller than the width, a ratio W1/T1 of the first width W1 to the first thickness T1 is at least 5 (five). This applies also to the second article 102, shown in Figs. 1b2 and 1c2. Thus, the second article 102 is made of wood based material. Moreover, the second article 102 has a second length L2, a second width W2, and a second thickness T2, wherein a ratio L2/T2 of the second length L2 to the second thickness T2 is at least 10 (ten) and a ratio W2/T2 of the second width W2 to the second thickness T2 is at least 5 (five).

Herein the term wood based material includes wooden veneers. Moreover the articles 101, 102 both have a volume of at least 10 cm³, typically much more, as will be detailed below. The articles 101, 102 are typically arranged in a pile, as opposed to e.g. a heap of sawdust or wood chips.

The articles 101, 102 are arranged into a pile, as shown in Fig. 1a. Generally in a pile, the articles are arranged on top of each other and such that the thicknesses of the articles are in the same direction. Thus, a direction of the first thickness T1 is unidirectional (i.e. parallel) with a direction of the second thickness T2. Moreover, typically the first thickness is unidirectional with a vertical direction Sz, as indicated in Fig. 1a. In Fig. 1a, the arrow G indicates a direction of gravity.

Having the articles 101, 102 arranged in a stable pile has the benefit that in such a case typically voids are not formed in the pile. Voids could cause the measurements of the moisture content being inaccurate. Thus, preferably, the pile comprising the first and second article 101, 102 comprises less than 20 vol% voids (i.e. holes). The term void refers to voids (i.e. holes) in between the articles. In addition, wood-based material may be porous, but the term "void" herein does not include porosity.

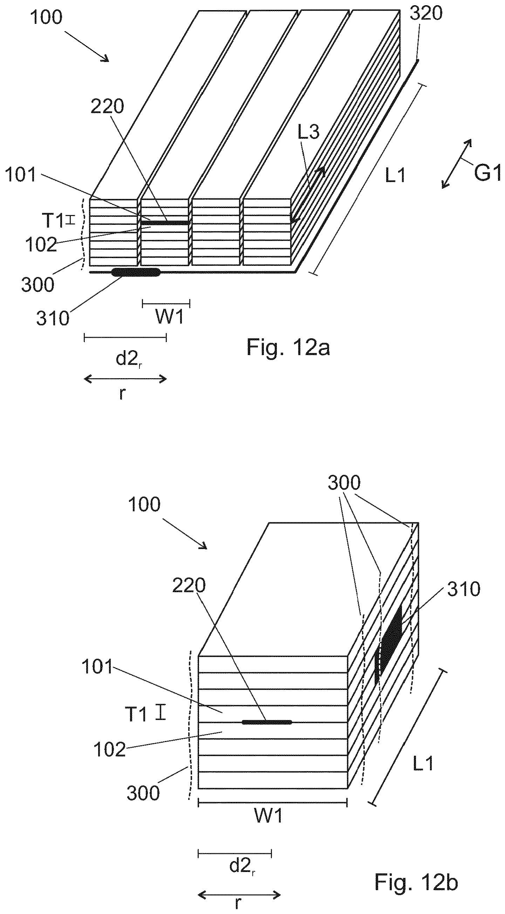

The length of the articles 101, 102 may be greater than their width, as indicated in Figs. 1b1 and 1b2. In the alternative, the length may be substantially equal to the width, as indicated in Figs. 1c1 and 1c2. Such articles are arranged on top of each other as indicated in Fig. 1a, 12a, and 12b. Moreover, in particular if the articles are narrow, similar articles may be arranged also side by side. Evidently, also wide articles could be arranged also side by side. However, in order to form a stable pile of the articles, the articles of a pile are substantially equally large. Therefore, in and embodiment, a ratio (L1/L2) of the first length L1 to the second length L2 is from 2/3 to 3/2. In the alternative, or preferably also, a ratio (W1/W2) of the first width W1 to the second width W2 is from 2/3 to 3/2. Even if articles of different thickness can be piled on top of each other in a stable manner, provided their lengths and/or widths are substantially the same (as defined above), typically also their thicknesses are substantially the same. Thus, in an embodiment, a ratio of the first thickness T1 to the second thickness T2 is from 2/3 to 3/2. Preferably these ratios apply to each pair of articles (101, 102, 103, 104, 105, 106) of the arrangement 100, wherein the articles are made from wood, excluding "dummy" pieces of wood. As indicated above, a prior art shipment may comprise such dummy pieces solely for the purpose of determining the moisture content. However, naturally an arrangement may comprise such dummy pieces for other purposes, too. More preferably, the ratio L1/L2 is from 4/5 to 5/4 and/or the ratio W1/W2 is from 4/5 to 5/4.

The arrangement 100 comprises a first remotely readable sensor device 211 configured to measure a value indicative of a moisture content. Figure 1d shows schematically some components of a sensor device 211. The sensor device 211 comprises a chip IC1, as shown in Fig. 1d. The chip IC1 is configured to provide information indicative of a moisture content of the environment of the chip IC1. Thus, when being arranged in between the first and second articles 101, 102, the chip IC1 is configured to provide information indicative of a moisture content of the first and second articles 101, 102. The chip IC1 may be or comprise a moisture content sensor. The chip IC1 may comprise various parts, including a radio frequency section and/or memory. In the alternative, the first remotely readable sensor device 211 may comprise separate circuit or circuits for those purposes. The first remotely readable sensor device 211 comprises an antenna Ant1 for transferring data. Optionally, the sensor device 211 comprises a source of electric energy (not shown). Because having the chip IC1, which is configured to provide information indicative of moisture content of the environment of the chip IC1, the first remotely readable sensor device 211 is configured to measure a value indicative of a moisture content. The term moisture content refers to water content of the environment wherein the sensor device 211 is. In particular the term moisture content refers to water content of the articles 101, 102, in between the which the sensor device 211 is arranged.

Preferably, the first remotely readable sensor device 211 is arranged in between the first and second articles 101, 102 such that first remotely readable sensor device 211 is in contact with the first article or the second article 102. As detailed below, in such a case, a part of a carrier 220 may be arranged in between the second 102 or the first 101 article respectively, whereby the first remotely readable sensor device 211 need not be in contact with the second 102 or the first 101 article respectively. Preferably, a distance between the chip IC1 and the first article 101 is at most 5 mm and a distance between the chip IC1 and the second article 102 is at most 5 mm. Also preferably, the first article 101 is adjacent to the second article 102. Thus preferably, a part of the first article 101 is in contact with a part of the second article 102.

Figure 1e shows in principle how the first remotely readable sensor device 211 configured to measure a value indicative of moisture content may be read with a reader device 910. A radio frequency signal 920 may be transmitted from the sensor device 211 to the reader device 910 or the signal may be otherwise readable by the reader device 910. E.g. in case an interrogator is used in the reader device 910, the sensor device 211 may modify an electromagnetic field generated by the reader device 910 in such a manner that the reader device may 910 read the signal from the modifications of the electromagnetic field, even if the sensor device 211 does not actively send a signal. Such solutions are known in the field of radio frequency identification (RFID). Referring to Fig. 1e, the first remotely readable sensor device 211 may be configured to draw electromagnetic energy from an electromagnetic field 920 generated by the reader device 910. In the alternative or in addition, the sensor device 211 may comprise a source of electric energy, such as a battery.

At least a part of the first remotely readable sensor device 211 is arranged in between the first article 101 and the second article 102. According to the present invention, at least a part of the chip IC1 is arranged in between the first article 101 and the second article 102. Moreover, the part of the first remotely readable sensor device 211 or the chip IC1 thereof is arranged in between the first article 101 and the second article 102 in the direction of the first thickness T1. Thus, the first article 101 and the second article 102 overlap with each other at least partly in the direction of the first thickness T1. In Fig. 1a, the direction Sz indicates a vertical direction, which is also unidirectional with the thicknesses T1 and T2 of the articles 101, 102. It has been found that a measurement result is much more accurate, when the first sensor device 211 is in between the two articles 101, 102 than in case the first sensor device 211 would be arranged e.g. on top of all the articles 101, 102. Therefore, the moisture content of the first and second article 101, 102 can be measured e.g. by the reader device 910, as indicated in Fig. 1e, and detailed above. This simplifies the procedure of measuring the moisture content of the articles 101, 102.

In addition, it has been found that the measurement results regarding moisture content are more reliable, when the chip IC1 of the first sensor device 211 is arranged suitably far away from the outer edges of the arrangement 100. Thus, in an embodiment, the first length L1 is at least 150 mm, and a first distance d1 in between the chip IC1 and an edge of the first article 101 is at least 50 mm. Herein the first distance d1 is measured in such a direction that the first distance d1 is unidirectional with the first length L1, as indicated in Fig. 1a. However, it has been found that the measurement results regarding moisture content are more reliable, when the chip IC1 is arranged at least 100 mm, more preferably at least 150 mm away from the outer edges of the arrangement 100. Thus, in an embodiment, the first distance d1 is at least 100 mm, more preferably at least 150 mm. In such cases, the first length L1 may be at least 200 mm or at least 300 mm, respectively.

Referring to Fig. 2, the arrangement 100 may comprise multiple (e.g. at least two, at least three, or at least four) remotely readable sensor devices (211, 212, 213, 214). In particular, in an embodiment, the arrangement 100 comprises a second remotely readable sensor device 212 configured to measure a value indicative of a moisture content, and a third remotely readable sensor device 213 configured to measure a value indicative of a moisture content. As indicated in Fig. 2 an embodiment further comprises and a fourth remotely readable sensor device 214 configured to measure a value indicative of a moisture content. What has been said about the internal structure and components of the first remotely readable sensor device 211 (see Fig. 1d), applies to the second, third, and fourth remotely readable sensor devices 212, 213, 214 *mutatis mutandis.* In particular each one of the remotely readable sensor devices (211, 212, 213, 214) comprises a chip configured to provide information indicative of a moisture content of an environment of the chip.

Using more sensor devices 211, 212, 213, 214 has the beneficial effect that statistics can be gathered. Moreover, since the first and second articles 101, 102 are made of wood based material, their moisture content is not necessarily uniform. In particular, the articles 101, 102 consist of wooden veneers, therefore these veneers may comprise notches, voids, or other irregularities affecting the measurement results. Thus, measurement accuracy can be improved by using more than one, preferably at least three, sensor devices 211, 212, 213, 214. Preferably, however, such sensor devices are arranged sufficiently far away from each other. Thus, in an embodiment, a distance between the chip IC1 of the first remotely readable sensor device 211 and a corresponding chip of the second remotely readable sensor device 212 is at least 50 mm (more preferably at least 100 mm); a distance between the chip IC1 of the first remotely readable sensor device 211 and a corresponding chip of the third remotely readable sensor device 213 is at least 50 mm (more preferably at least 100 mm); and a distance between a chip configured to provide information indicative of a moisture content of an environment of the second remotely readable sensor device 212 and a chip configured to provide information indicative of a moisture content of an environment of the third remotely readable sensor device 213 is at least 50 mm (more preferably at least 100 mm). In case the arrangement comprises the fourth sensor device 214, distances between a corresponding chip of the fourth sensor device 214 and the corresponding chip of the other sensor devices (211, 212, 213) are at least 50 mm, preferably at least 100 mm. Such distances ensure that the different sensors 211, 212, 213, 214 are not measuring moisture content from the same part of the arrangement, whereby the obtainable data represents moisture content in different locations. As indicated above, some of these locations may have irregularities.

In a preferable embodiment, the arrangement 100 comprises such an amount of such sensor devices 211, 212, 213, 214, that the moisture content can be measured with an accuracy of +-1 % unit or better. A typical moisture content (i.e. water content) may of the order of 5 %, in which case 1 % unit would correspond to 20 percentage. Typically three sensor devices suffice, and a fourth can improve accuracy. The type of the chip IC1 used to sense the moisture content may also affect the number of sensors needed for high accuracy. In addition, temporal averaging of signals from each sensor can be used to improve accuracy, as well known in the art of measuring. Moreover, typically a chip IC1 detects moisture content of surroundings of the chip IC1, whereby the measurement is naturally an average over some parts of the first and second articles 101, 102 close to the chip IC1. This also improves measurement accuracy.

Moreover, preferably when the arrangement 100 comprises at least two sensor devices 211, 212, such as at least three sensor devices 211, 212, 213 or four sensor device, all these sensor devices (211, 212, optionally also 213 or both 213 and 214) are readable by the same reader device 910. This has the technical effect that statics can be taken into account already in the same reader device 910. Thus, the user of the reader device 910 obtains a more accurate result without further processing in another device.

Referring to Fig. 3, the arrangement 100 comprises a wrapping 300. The wrapping 300 surrounds the first article 101, the second article 102 and the first remotely readable sensor device 211. Preferably, the wrapping 300 surrounds an arrangement of the articles (101, 102, ..., 106) and the sensor devices (211, 212, 213, 214). As indicated above, preferably, the first sensor device 211 is arranged suitably far away from the edges of the arrangement 100. This applies also, when the arrangement comprises multiple piles of articles side by side, as in Fig. 12a. Thus, even if the chip IC1 is not necessarily far from a side of the first article 101 (as measured in a direction of the first width W1), the chip IC1 may be far from the wrapping 300, even if the wrapping contacts all the articles of the arrangement. In Fig. 12a, only a carrier 220 is shown. At this point it suffices to note that the first sensor device 211 having the chip IC1 may be fixed to the carrier 220. Therefore, in an embodiment, all second distances d2ᵣ between the chip IC1 and the wrapping 300 are at least 50 mm. Herein a second distance d2ᵣ is a distance between the chip IC1 and the wrapping 300, as measured in a direction r. Figure 3 shows, as an example, two such second directions, of which a primary is measure in the direction of thickness and a secondary in the direction of length. Figures 12a and 12b show this distance, when the direction is unidirectional with the width and the chip (IC1) is substantially at a centre of the carrier 220. Figures 12a and 12b show only a part of the wrapping 300. In case the arrangement comprises further sensor device 212, 213, 214 and the wrapping 300, preferably the wrapping 300 surrounds also the other sensor devices; in particular the second 212 and third 213. The wrapping 300 comprises plastic material. Preferably, all second distances d2ᵣ between the chip IC1 and the wrapping 300 are at least 100 mm, and even more preferably at least 150 mm. In case the arrangement comprises further sensor devices 212, 213, 214, this distance applies also to their chips configured to provide information indicative of a moisture content (i.e. water content) *mutatis mutandis.*

The wrapping 300 has at least the technical functions of protecting the articles 101, 102 and binding the articles 101, 102 together. Moreover, typically the articles 101, 102 are relatively dry, whereby another function of the wrapping 300 may be to protect the articles from humidity and/or water. For example, an average moisture content of the first article 101 and the second article 102 may be from 1 % to 15 %, such as from 1.5 % to 10 %, more preferably from 2 % to 9 %. In such a case many environments are so humid, that the articles 101, 102 tend to absorb moisture from the environment.

In order to protect the articles from high or low humidity (either from moisturizing or from drying), in an embodiment, the wrapping 300 is made of material that is diffusion closed. A material that is diffusion closed has an Sd value of at least 0.5 m, when measured as indicated in the standard ISO 12572 (2001). More preferably, an Sd value of the wrapping 300 is at least 1 m such as at least 5 m. The wrapping 300 comprises plastic and is used to prevent wetting and drying.

In an embodiment, the wrapping 300 is also waterproof. The waterproofness of the wrapping 300 can be characterized e.g. by the IP level 2. The IP code in the IP level refers to the Ingress Protection Rating, i.e. International Protection Rating. It is defined in the standard ANSI/IEC 60529-2004 (approved November 3, 2004). Thus, the wrapping 300 may be waterproof against water equivalent to 3 mm rainfall per minute, at least for 10 minutes.

Another possibility to characterize waterproofness is to assume that wetting through the wrapping 300 occurs by Fickian diffusion. For example, the diffusion coefficient for some polymers that are generally considered water proof are about 3×10⁻¹² m²/s. Preferably, the diffusion coefficient for water of the material of the wrapping 300 is less than 1×10⁻⁶ m²/s. More preferably, the diffusion coefficient of the material of the wrapping 300 is less than 1×10⁻⁹ m²/s. It is noted that the diffusion coefficient is dependent on temperature, and the given limit refers to normal operating temperature of the panel. The normal operating temperature may be e.g. 30 °C. Measurement of diffusion coefficient is disclosed e.g. in a document "Absorption and Diffusion of Moisture In Polymeric Materials" by Duncan and Boughton (National Physical Laboratory), ISBN 1368-6550. Typically, the diffusion coefficient for water in a plastic material may range e.g. from 7×10⁻¹⁴ m²/s as in Polyamide-6 to 2.5×10⁻¹⁰ m²/s as in poly vinyl chloride, a value for e.g. polyethylene lying in between these values.

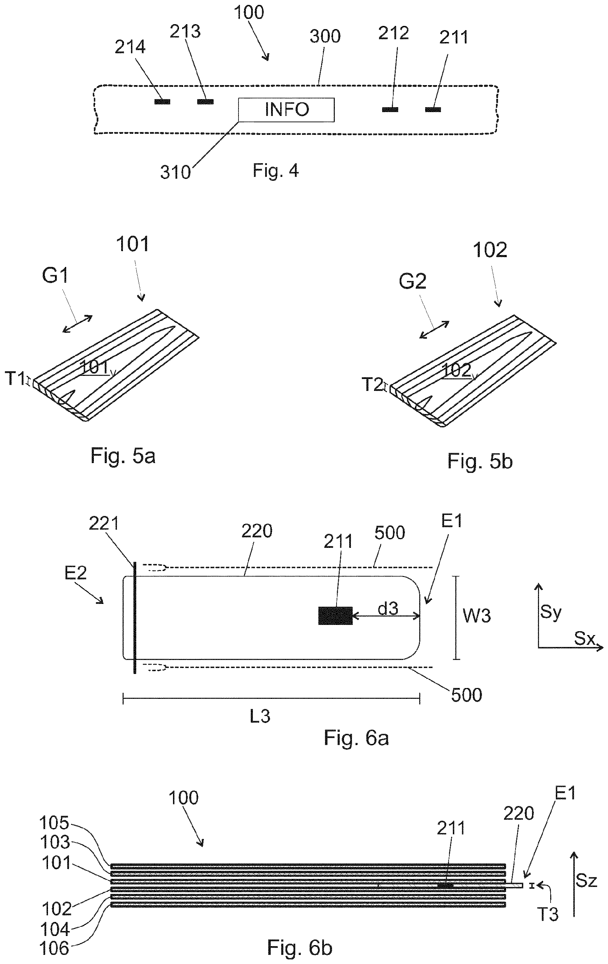

In a preferable embodiment, the arrangement 100 comprises an information carrier 310 comprising information INFO indicative of an identity of the first remotely readable sensor device 211. The information carrier 310 may be arranged e.g. on the wrapping 300 (as in Fig. 4) or the information carrier may be arranged e.g. on a pallet 320 on which the articles are piled, as in Fig. 12a. Possible other locations for the information carrier 310 include in between the articles and the wrapping 300 at least when the wrapping is transparent as indicated in Fig. 12b. Such a position is often preferable, at least when the wrapping is transparent and waterproof. In such a case the information carrier 310 may be read optically, and may comprise a piece of paper since the wrapping 300 protects the information carrier 310. The information carrier 310 may be a piece of paper or sticker printed with the information (e.g. bar code or QR code or the identity of the first sensor device 211). The information carrier 310 may be an NFC (near field communication) circuit. The information carrier 310 may be an UHF RFID tag. Herein "F" in UHF refers to the frequency of a communication signal of the RFID, and "UH" refers to ultra high. Moreover, UHF indicates that this frequency is from 300 MHz to 3 GHz.

As for a background of the technical function of the information carrier 310, in a warehouse, several similar arrangements may be present, each having at least one, preferably at least three sensor devices. All these sensor devices may be remotely readable by the same reader device 910. As all these sensor devices are remotely readable by the same device 910, it is hard to know, which one(s) of the readable signals are from the arrangement 100, of which moisture content is to be measured. In particular, some of the signals may originate from sensor devices of other arrangements. When the information carrier 310 comprises information INFO indicative of an identity of the first remotely readable sensor device 211, this information can be used to pick only the correct signal and/or to activate only the first remotely readable sensor device 211 for reading the moisture content. When the arrangement 100 comprises also other sensor device(s) 212, 213, 214, preferably the information carrier 310 comprises information INFO that is also indicative of the identity/identities of the other sensor devices (212, 213, 214). In particular, in an embodiment, the arrangement comprises at least the first, second, and third remotely readable sensor devices 211, 212, 213 and the information carrier 310 comprises information INFO indicative of an identity of the first remotely readable sensor device 211, an identity of the second remotely readable sensor device 212, and an identity of the third remotely readable sensor device 213. Preferably the information carrier is arranged on the wrapping 300, as indicated in Fig. 4, or between the wrapping 300 and the articles, as indicated in Fig. 12b. In an embodiment, the arrangement further comprises the fourth remotely readable sensor device 214 and the information carrier 310 further comprises information INFO indicative of an identity of the fourth remotely readable sensor device 214.

As for the type of the articles, according to the present invention, the first article 101 is a first wooden veneer 101v, as indicated in Fig. 5a. In a similar manner, the second article 102 is a second wooden veneer 102v. In general, a wooden veneer 101v, 102v has a grain orientation G1, G2, which is unidirectional with a length of a log from which the wooden veneer has been manufactured, wherein the length of the log is the upwards vertical direction, when the tree, from which the log has been cut, grows. Typically in a log, annual rings encircle axes that are unidirectional with the grain orientation G1, G2. Correspondingly, in a veneer, the traces of the annual rings extend substantially parallel with the grain orientation, as shown in Figs. 5a and 5b. Examples of wood based articles that comprise or consist of a wooden veneer (101v, 102v) include: the wooden veneer itself, a piece of plywood, and a flooring board comprising a top and a bottom veneer. In general, a thickness of a wooden veneer (101v, 102v) is from 0.5 mm to 5 mm, such as from 0.5 mm to 4 mm. A plywood board comprising such a veneer has a greater thickness, which depends on the number of veneers in the plywood board.

Such articles are preferably arranged into a pile such that the first grain orientation G1 is unidirectional with the second grain orientation G2. In such a case, the first sensor device 211 can be slid to its place in the grain direction G1, G2 relatively easily.

A method for forming the arrangement 100 comprises receiving a first article as discussed above, the second article 102 as discussed above, and arranging the first article 101 and second article 102 relative to each other such that the first thickness T1 is unidirectional with the second thickness T2, and such that at least a part of the first article 101 overlaps in a direction of the first thickness T1 with at least a part of the second article 102. The method further comprises arranging at least a part of the chip IC1 of the first remotely readable sensor device 211, which is configured to measure a value indicative of a moisture content, in between the first article 101 and the second article 102 in the direction of the first thickness T1.

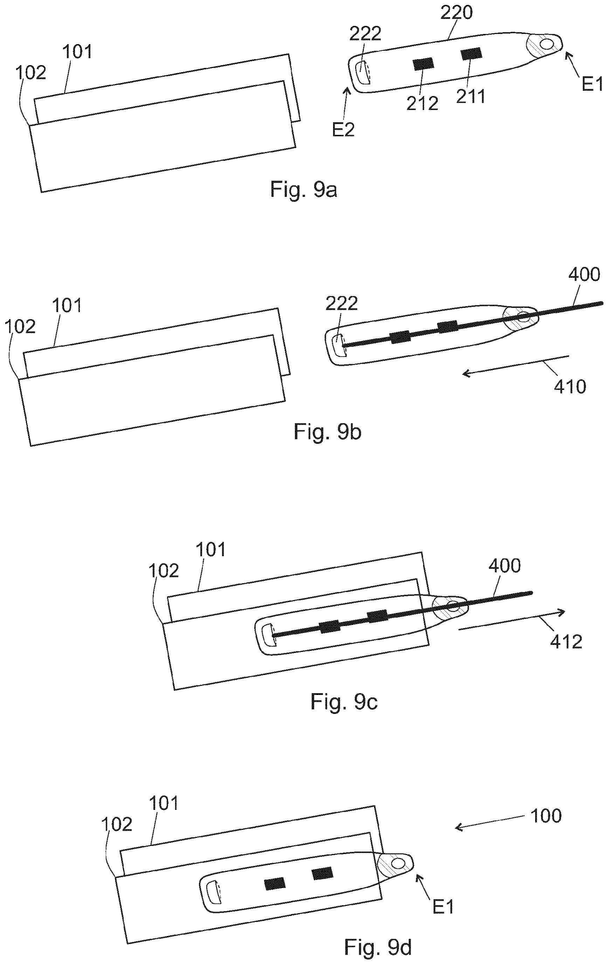

As for the order of these steps, it is possible that the articles 101, 102 are arranged on top of each other first, and thereafter, at least a part of the chip IC1 of the first remotely readable sensor device 211 is arranged in between the articles. Such method is disclose in Figs. 9a to 9d.

In the alternative, it is possible to arrange the first article 101 to a location. Thereafter, arrange at least a part of the chip IC1 of the first remotely readable sensor device 211 on top of the first article 101. And only thereafter, arrange the second article 102 on top of the first article 101 such that (i) the first thickness T1 is unidirectional with the second thickness T2, (ii) at least a part of the first article 101 overlaps in a direction of the first thickness T1 with at least a part of the second article 102, and (iii) at least a part of the chip IC1 of the first remotely readable sensor device 211 that is configured to measure a value indicative of a moisture content becomes arranged in between the first article 101 and the second article 102 in the direction of the first thickness T1. Thus, a pile may be built in the following order: the first article 101, the first remotely readable sensor device 211, and the second article 102.

In comparison to prior art, the arrangement 100 has several beneficial effects, including:
- possibility to obtain instant measuring result,
- labour cost savings,
- automatic data transfer,
- no material loss,
- no need to open wrapping (i.e. package),
- possibility to measure same package several times,
- possibility to measure an average moisture content of several articles (depending on the type of the chip IC1), which improves accuracy.

For example, if a moisture would be determined by drying a sample of the shipment, this would amount to considerable material loss in the long run.

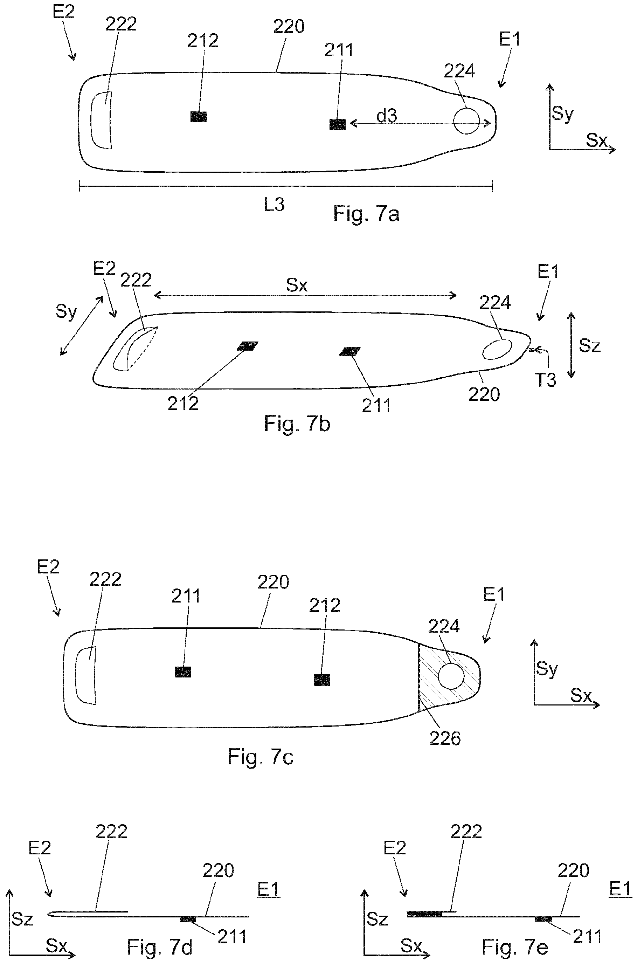

Referring to Figs. 6a and 7a, in order to apply the first sensor device 211 to a proper location within the arrangement, it has been noticed that a carrier 220 can be used for that purpose. Figs. 6a and 7a show embodiments of such a carrier 220 in a top view. Fig. 7b shows the embodiment of Fig. 7a in a perspective view.

Referring to Figs. 6a and 7a, in an embodiment of the arrangement 100 and in a method for forming the arrangement 100, the the first remotely readable sensor device 211 is fixed to a carrier 220. The carrier 220 may be made of suitable material. For example, the carrier 220 may be made of wood. Even if wood absorbs moisture, the moisture content thereof will equalize with the moisture content of the articles 101, 102, whereby this material will not deteriorate the measurements. In the alternative, the carrier may be made from polymer material. In general, polymer materials absorb only a little moisture, whereby also they do not deteriorate the measurements. Moreover, neither wood, nor polymers significantly affect the RF communication in between the sensor device 211 and a reader device 910. Moreover, the carrier 220 may be made from the same material as the first article 101. In particular, one of the articles 101, 102, 103, 104, 105 may serve as the carrier 220. Preferably, the carrier 220 is made of electrically insulating material. This has the effect that the antenna Ant1 of the sensor device 211 is sensitive to electromagnetic radiation. Consequently, in an embodiment, the carrier comprises material of which electrical resistivity is at least 10 Ωm at a temperature of 23 °C. Preferably, the sensor device 211 is fixed to the carrier 220 at such a location, wherein an electrical resistivity of the material of the carrier 220 is at least 10 Ωm at a temperature of 23 °C.

The carrier 220 has a third length L3 of at least 150 mm in between a first end E1 of the carrier 220 and a second end E2 of the carrier 220. This has the effect that the chip IC1 can be placed sufficiently far away from an edge of the first article, even if a part of the carrier is not arranged in between the first and second articles. Preferably, the carrier 220 has a third width W3 of at least 50 mm. This is beneficial at least when the carrier 220 comprise a holder, as detailed below. A narrower carrier 220 would not have sufficient space for a holder.

Moreover, a thickness of the carrier 220, i.e. a third thickness T3, is reasonably small. On one hand this ensures that the carrier 220 is somewhat flexible, whereby voids (see above) can be avoided. On the other hand, this ensures that large void will neither be present near the edges of the carrier 220. Thus, in a preferable embodiment, the third thickness T3 (of the carrier) not greater than the first thickness T1 (of the first article 101). The third thickness T3 does not include the thickness holder 221, 222 (if present) or the sensor device(s) 211, 212. Moreover, the first sensor device 211 is fixed to such a location of the carrier 220 that a third distance d3 between the first end E1 of the carrier and the chip IC1 of the first remotely readable sensor device 211 is at least 100 mm. This has the effect that the chip IC1 can be placed sufficiently far away from an edge of the first article, even if a part of the carrier is not arranged in between the first and second articles.

In an embodiment of the arrangement 100 at least a part of the carrier 220 is arranged in between the first article 101 and the second article 102 in the direction of the first thickness T1. An embodiment of the method comprises arranging at least a part of the carrier 220 in between the first article 101 and the second article 102 in the direction of the first thickness T1. Preferably, also the second remotely readable sensor device 212 configured to measure a value indicative of moisture content is fixed to the carrier 220. This applies both to the arrangement 100 and the method. This is shown in the embodiment of Figs. 7a and 7b, but can apply to another type of a carrier 220 (e.g. that of Fig. 6a).

When the first remotely readable sensor device 211 is fixed to the carrier 220, preferably, the carrier 220 is in contact with the first article 101 or the second article 102. More preferably, the carrier 220 is in contact with the first article 101 and the second article 102.

In a preferable embodiment, the carrier 220 comprises a holder 221, 222. The holder 221, 222 is configured to receive a part of a tool (e.g. the tool 500 of Fig. 6a or the or rod 400 of Figs. 9a-d) for inserting at least a part of the carrier 220 in between the articles 101, 102. Referring to Fig. 6a, the holder 221 may have a shape of a bar that protrudes in a direction of a width of the carrier. Thus, the insertion tool 500 may comprise e.g. Y -shaped branches, as shown in Fig. 6a, that are configured to match the bar (i.e. the holder 221) while inserting the carrier 220 by pushing the carrier 220 from the holder 221. A distance between the holder 221, 222 and the second end E2 of the carrier 220 is less than a distance between the holder 221, 222 and the first end E1 of the carrier 220. The holder 221, 222 may be arranged at the second end E2 of the carrier 220, as indicated in Figs. 6a, 7a and 7b.

Referring to Figs. 7a and 7b, in a preferable embodiment of the arrangement 100 and the method for forming the arrangement 100, the carrier 220 comprises such a holder 222 that is closed towards the second end E2 of the carrier 220 and open towards the first end E1 of the carrier 220. The holder 222 may be e.g. a fold of the carrier 220, as indicated in Fig. 7d. The sides of the fold (which run in the direction Sx of Fig. 7d) may be closed e.g. by glue. The holder 222 may be e.g. a pouch glued onto the carrier 220, as indicated in Fig. 7e. For example, a piece of sheet like material may be glued onto the carrier 220 from the second end E2 and the sides which run in the direction Sx of Fig. 7e, whereby a pouch is formed. As indicated in Figs 7d and 7e, and also in Fig. 7b, the holder 222 is open towards the first end E1. A reinforcement (not shown) may be provided at the second end E2 of the carrier 220 for reinforcing the holder 222. The reinforcement may improve the re-usability of the carrier, since most of the stress caused by the tool or rod (400, 500) concentrates on the holder 222.

Figures 9a to 9d show an embodiment of a method for forming the arrangement 100, when a carrier 220 comprising the holder 222 is used. In that embodiment, the first remotely readable sensor device 211 is arranged on the carrier 220, of which properties have been disclosed above. Fig. 9a shows the articles 101, 102 and the carrier 220 comprising the first sensor device 211. Referring to Fig. 9b, the embodiment comprises arranging a part of a rod 400 into the holder 222. The rod 400 can be used to push the carrier 220 from the holder 222, because the holder 222 is closed towards the second end E2. The arrow 410 in Fig. 9b indicates the direction of pushing the carrier 220 by the rod 400. Thus, the embodiment comprises by using the rod 400, pushing at least a part of the carrier 220 in between the first article 101 and the second article 102 such that at least a part of the first remotely readable sensor device 211 (in particular at least part of the chip IC1) becomes arranged between the first article 101 and the second article 102 in the direction of the first thickness T1. In this way, the chip IC1 becomes arranged in a proper location. Figure 9c shows the arrangement after having pushed the carrier 220 to its place. For clarity, the second article 102 is shown as being transparent in Fig. 9c.

However, the arrangement 100 is typically a shipment, whereby the rod is typically not a part of the shipment. Therefore, preferably the embodiment further comprises pulling the rod 400 from the holder 222 in such a way that at least a part of the chip IC1 of the first remotely readable sensor device 211 remains between the first article 101 and the second article 102 in the direction of the first thickness T1. The arrow 412 shows the direction to which the rod 400 can be pulled. Fig 9d shows the resulting arrangement 100. For clarity, the second article 102 is shown as being transparent in Fig. 9c. In 10a the second article 102 is shown as opaque. In general wood based materials are opaque.

A part of the rod 400 can be arranged into holder 222 and removed therefrom, since the holder 222 opens towards the first end E1.

The tool 500 of Fig. 6a can be used in a similar manner to push to carrier 220 to its place within the arrangement 100.

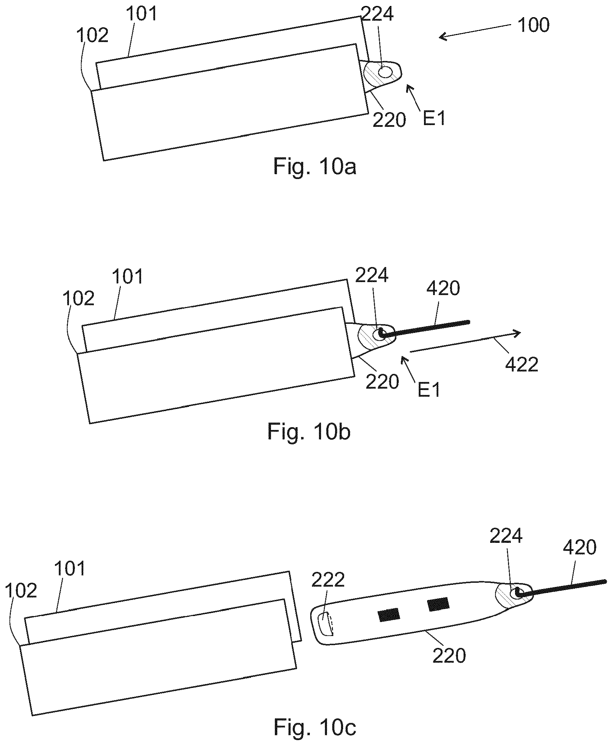

Fig. 10a shows an arrangement 100, wherein the first end E1 of the carrier is not arranged in between the first article 101 and the second article 102 in the direction of the first thickness T1. A method for forming such an arrangement comprises pushing at least a part of the carrier 220 in between the first article 101 and the second article 102 such that, after said pushing, the first end E1 of the carrier 220 protrudes from an edge of the first article 101 or the second article 102. Because the first end E1 protrudes in such a manner, the carrier 220 is removable from the arrangement 100 by pulling from the first end E1.

When the articles 101, 102 are used, the sensor device 211 is not further needed. Thus, the carrier 220 may also be removed. The carrier 220 may also be re-used as part of another arrangement. Referring to Figs. 10b and 10c, the carrier 220 may be removed by using a grabbing means. For example the grabbing means may be a hook 420 or a finger of a user. The grabbing means may be used to grab the carrier 220 from the first end E1 thereof. Then the carrier 220 can be pulled from the arrangement 100.

In order to ease the removal of the carrier 220, in an embodiment the carrier limits an aperture 224. The aperture 224 is preferably arranged at the first end E1 of the carrier 220. Such an aperture 224 is shown in Figs. 7a to 7c, 8, 9a to 9d, and 10a to 10c. Moreover, in order to help to find the carrier 220 from the arrangement 100, in an embodiment, the first end E1 of the carrier 220 is not transparent. Naturally, the first end E1 need not be completely opaque. The first end may be coloured to have an easily noticeable colour, such as a colour corresponding to a wavelength of from 700 nm to 800 nm (i.e. red or orange). The first end E1 being not transparent is indicated by hatching in Figs. 7c, 8, 9a to 9d, and 10a to 10c.

After arranging at least part of the chip IC1 in between the articles 101 and 102 as discussed above, the arrangement comprising the articles 101 and 102 and the sensor device 211 is wrapped to form an arrangement further comprising the wrapping 300. Thus, the method comprises, after arranging at least a part of the chip IC1 in between the first article 101 and the second article 102, wrapping a combination comprising the first article 101, the second article 102, and first remotely readable sensor device 211. In an embodiment, the first article 101, the second article 102, and first remotely readable sensor device 211 are wrapped by a wrapping 300 such that not even a part of the wrapping 300 is arranged in between (i) the first article 101 and the second article 102, (ii) in between the first article 101 and the first remotely readable sensor device 211, or (iii) in between the second article (102) and the first remotely readable sensor device. In case the arrangement comprises also other sensor device 212, 213, 214, also these may be surrounded by the wrapping. I.e. an arrangement comprising also this/these other sensor device(s) but without the wrapping 300 may be wrapped to form an arrangement having the wrapping 300.

In a preferably embodiment, of the arrangement 100 and the method, the first article 101 consists of or comprises a first wooden veneer 101v having a first grain orientation G1 and the second article 102 consists of or comprises a second wooden veneer 102v having a second grain orientation G2 such that the first and second grain orientations G1, G2 are unidirectional.

A preferable embodiment of the method comprises, by moving the rod 400 in the first grain orientation G1, pushing at least a part of the carrier 220 in between the first article 101 and the second article 102 such that at least a part of the chip IC1 of the first remotely readable sensor device 211 becomes arranged between the first article 101 and the second article 102 in the direction of the first thickness T1. This helps inserting the carrier 220, since the carrier is easier to move in the first grain orientation G1 than in another orientation. In this embodiment, preferably, the carrier 220 is in contact with both the first article 101 and the second article 102. In other words, no other wood based article (103, 104, 105, 106) is arranged in between the carrier 220 and the first article 101, and no other wood based article (103, 104, 105, 106) is arranged in between the carrier 220 and the second article 102. More preferably, the carrier 220 is in contact with the wooden veneer 101v of the first article or the wooden veneer 102v of the second article 102. More preferably, the carrier 220 is in contact with the wooden veneer 101v of the first article 101 and the wooden veneer 102v of the second article 102. As indicated above, preferably, the grain orientations G1 and G2 of the wooden veneers 101v and 102v are substantially unidirectional. The rod 400 need not be moved precisely in the grain orientation. The direction of movement of the rod may form an angle of at most 15 degrees or at most 5 degrees with the first grain orientation G1.

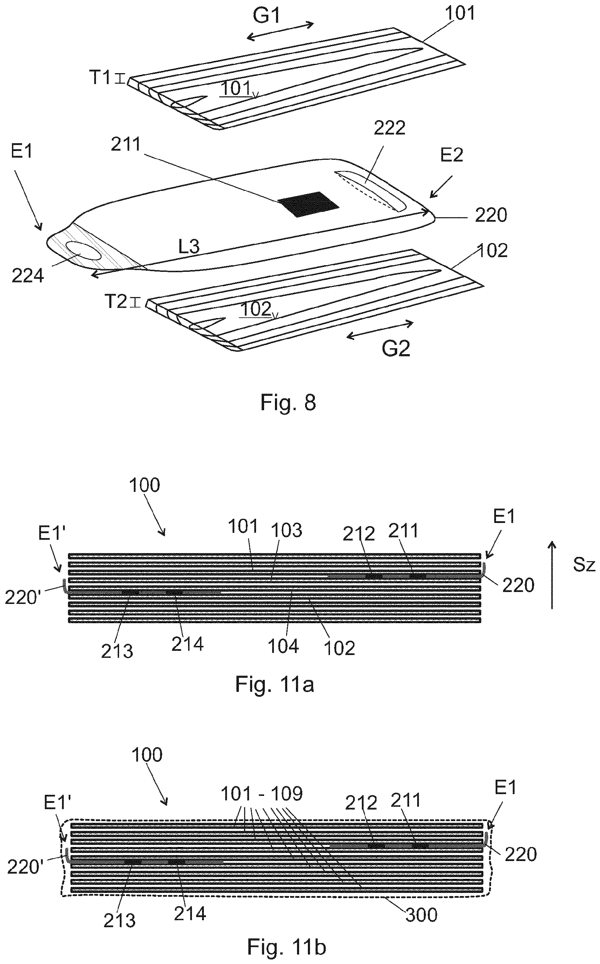

With reference to Fig. 8, in a corresponding arrangement 100, a direction of the third length L3 forms an angle of at most 15 degrees with the first grain orientation G1 or is unidirectional with the first grain orientation G1. Preferably, also in the arrangement 100, the carrier 220 is in contact with both the first article 101 and the second article 102. In other words, no other wood based article (103, 104, 105, 106) is arranged in between the carrier 220 and the first article 101 and no other wood based article (103, 104, 105, 106) is arranged in between the carrier 220 and the second article 102. More preferably, the carrier 220 is in contact with the wooden veneer 101v of the first article or the wooden veneer 102v of the second article 102. More preferably, the carrier 220 is in contact with the wooden veneer 101v of the first article 101 and the wooden veneer 102v of the second article 102. Such an embodiment is depicted in Fig. 8 (however, for reasons of illustration, the articles 101 and 102 and the carrier 220 are not drawn to be in contact with each other, even if, in a pile, they would be).

Regarding typical sizes of the articles, in an embodiment, the first length L1 is at least 600 mm, the first width W1 is at least 40 mm, and the first thickness T1 is from 0.5 mm to 30 mm. As an example, the first length L1 may be from 600 mm to 4000 mm. As an example, the first width W1 may be from 40 mm to 450 mm. The aforementioned range of the first thickness T1 applies to individual veneer. However, in case the articles are individual veneers, the first thickness T1 may be e.g. from 0.5 mm to 4 mm.

Preferably, the first sensor device 211 and the second sensor device 212 is fixed to the carrier 220, i.e. a first carrier 220, as shown in Fig. 11a. Moreover a second carrier 220', which also comprises two sensor device, i.e. the third sensor device 213 and the fourth sensor device 214, is comprised by the arrangement 100. Thus, an arrangement comprises a first carrier 220 and a second carrier 220', each one of the carriers 220, 220' comprising two sensor devices. A part of the first carrier 220 (in particular at least parts of the sensor devices 211, 212 fixed to the first carrier 220) are arranged in between the first article and the second article. However, the first end E1 of the first carrier 220 is not arranged in between these articles to help removal of the first carrier as detailed above. A part of the second carrier 220' (in particular at least parts of the sensor devices 213, 214 fixed to the second carrier 220') is arranged in between the first article and the second article. However, the first end E1' of the second carrier 220' is not arranged in between these articles to help removal of the second carrier 220' as detailed above.

At least a part of the second carrier 220' may be pushed in between two articles in the same way as the first carrier 220 is pushed in between two articles, *mutatis mutandis.* Thus, even if not shown, preferably also the second carrier 220' comprises a holder corresponding to the holder 222 of the first carrier 220.

As indicated in Fig. 11a, the different sensor devices 211, 212, 213, 214 need not be at the same vertical level. As indicated in Fig. 11a, the arrangement may comprise a third article 103 such that a part of the first carrier 220 is arranged in between the first article 101 and the third article 103, but not even a part of the second carrier 220' is arranged in between the first article 101 and the third article 103. However, a part of the second carrier 220' may be arranged in between the second article 102 and the third article 103, even if not even a part of the first carrier 220 is arranged in between the second article 102 and the third article 103. Fig. 11a shows also a fourth article 104, wherein the third article 103 and the fourth article 104 are arranged in between the first and second articles. The arrangement of Fig. 11a shows nine articles. However, typically an arrangement 100 comprises tens or several tens of articles. It is also noted that the articles may be numbered arbitrarily. Moreover, in Fig. 11a, the first carrier 220 is arranged in between two adjacent articles (101, 103) and the second carrier 220' is arranged in between two adjacent articles (104, 102) in the meaning discussed above. As indicated in Fig. 11b, the arrangement 100 may comprise a wrapping 300. Also the first end E1 of the first carrier 220 may be surrounded by the wrapping 300. When the arrangement comprises the second carrier 220', also the first end E1' of the second carrier 220' may be surrounded by the wrapping 300.

As indicated above, an referring to Fig. 7c, in an embodiment, the first end E1 of the carrier 220 is preferably not transparent. This has the effect that a user can easily see a location of the carrier 220. However, the rest of the carrier may be transparent or non-transparent. In case the whole carrier is not transparent, e.g. painted with the same colour, the carrier preferably comprises a marking 226 indicative of a preferable location of an end of an article 101, 102. Thus, when pushing the carrier 220 in between the articles 101, 102, as detailed above, the marking 226 can be aligned with an end of one of the articles 101, 102, whereby the first sensor device 211 will be aligned sufficiently deep into the arrangement, as detailed above. Moreover, then a sufficiently long portion of the carrier will protrude from the end(s) of the articles 101, 102 for the removal of the carrier 220. In case the second end E2 of the carrier 220 is transparent, and the first end E1 is not transparent, a limit between these areas can be considered to form the marking 226. Even is such a case, an additional marking can be used. When the marking 226 and an end of an article 101, 102 is aligned as indicated above, a distance between the marking 226 and an end of one of the first article 101 and the second article 102 is at most 75 mm, such as at most 50 mm. This concerns an embodiment of an arrangement 100, and in a corresponding method the carrier 220 is aligned relative to an end of an article 101, 102 in such a manner.

Referring to Figs 7d and 7e, in an embodiment, the holder 222 is arranged on a first side of the carrier 220, and the first sensor device 211 is arranged on a second side of the carrier 220, wherein the second side is opposite to the first side. In case the second sensor device 212 is fixed to the carrier 220, also the second sensor device 212 may be arranged to the second side of the carrier 220. This may improve the reliability of the carrier, since when the rod 400 is used to handle the carrier, the rod 400 may also hit the sensor device(s) 211, 212. However, when the rod is only used on the first side of the carrier 220 (when the holder 222 is on the first side), and the sensor devices(s) 211, 212 are on the opposite side, the rod 400 only glides on the carrier 220, by does not hit the sensor devices 211, 212. Thus, the reliability is improved.

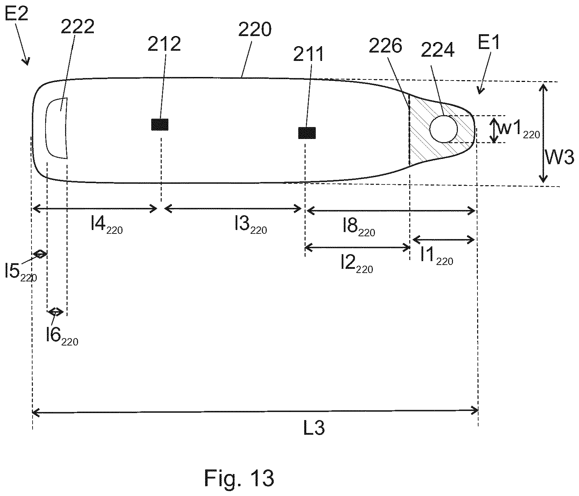

Some preferable measures for the carrier 220 are discussed below with reference to Fig. 13. A length l1₂₂₀ between the first end E1 and the marking 226, e.g. a length of the non-transparent area, as measured from the first end E1 of the carrier 220, may be e.g. at least 70 mm, such as 70 mm - 200 mm, such as at least 100 mm or 100 mm - 150 mm. The lower limit ensures that the non-transparent area is sufficiently large for it to be visually easily detectable.

A length l2₂₂₀ in between the marking 226 of the carrier 220 and the chip IC1 of the first sensor device 211 may be e.g. 50 mm - 300 mm, such as 150 mm - 250 mm. The lower limit ensures that the chip may be sufficiently deep in the arrangement, even if the first end E1 is not arranged in between the articles. As indicated above, this is beneficial for the removal of the carrier 220. The upper limit is beneficial for material costs.

The length l8₂₂₀ is the sum of the length l1₂₀₀ and l2₂₀₀. However, also this length is presented separately, since the definition of the lengths l1₂₀₀ and l2₂₀₀ requires the presence of the marking 226, and the marking is not present in all embodiments. The length I8₂₂₀ in between the first end E1 of the carrier 220 and the chip IC1 of the first sensor device 211 may be e.g. 250 mm - 500 mm, such as 300 mm - 400 mm. The lower limit ensures that the chip may be sufficiently deep in the arrangement, even if the first end E1 is not arranged in between the articles. As indicated above, this is beneficial for the removal of the carrier 220. The upper limit is beneficial for material costs.

A length l3₂₂₀ in between the chip IC1 of the first sensor device 211 and a chip of the second sensor device 212 may be e.g. 100 mm - 200 mm, such as 125 mm - 175 mm. The lower limit ensures that the chips are sufficiently far from each other to properly measure the moisture content of different locations. The upper limit is beneficial for material costs.

A length l4₂₂₀ in between the chip IC1 of the second sensor device 212 and the second end E2 may be e.g. 70 mm - 250 mm, such as 100 mm - 200 mm.

The lower limit ensures that the holder 222 has sufficient space and the upper limit is beneficial for material costs.

In Fig. 13, the holder 222 is closed towards the second end E2 and open towards to first end E1, as discussed above.

A length I5₂₂₀ in between second end E2 and a closed end of the holder 222 may be e.g. 3 mm - 50 mm, such as 50 mm - 15 mm. The lower limit ensures that closed end of the holder 222 has sufficient strength for receiving the rod 400 while inserting the carrier. The upper limit is beneficial for inserting the carrier 220, since this measure ensures the holder 222 is sufficiently close to the second end E2. If the holder 222 is formed as fold (see e.g. Fig. 7d), the length I5₂₂₀ may be smaller, and may correspond to a thickness of the carrier 220.

A length l6₂₂₀ in between the closed end of the holder 222 and the open end of the holder 222 may be e.g. 20 mm - 100 mm, such as 25 mm - 50 mm. The lower limit ensures that an end of the rod 400 can be reliably received by the holder 222. The upper limit is beneficial for material costs.

In this way, the length L3 of the carrier 220 may be e.g. 300 mm - 750 mm. It is also noted that the carrier 220 need not to be fixed to the second sensor device 212, in which case the carrier may be shorter than in case two sensor device 211, 212 are fixed thereto. Moreover, in case three sensor devices 211, 212, 213 are fixed to the carrier 220, the carrier 220 may be longer than in case it is fixed to only two sensor devices. The distance l3₂₂₀ disclosed above indicates how adding or removing sensor devices 213, 212 from the carrier 220 may affect the length requirement for the carrier. When only two sensor devices 211, 212 are fixed to the carrier 220, as in Fig. 13, the length L3 of the carrier 220 is preferably from 400 mm to 750 mm, more preferably from 500 mm to 700 mm. The lower and upper ends are preferable for the technical functions as indicated above. Moreover, in case two carriers are used in an arrangement 100, and applied from opposite ends of the arrangement, as in Figs. 11a and 11b, it is also beneficial that the length of such a part of the carrier 220 that is arranged between the articles is at most half of a length L1 of the articles. This ensures that the sensor devices of different carriers 220, 220' do not overlap in the direction of thickness. As for the length of the part remaining in between articles, preferably, the non-transparent area, and only the non-transparent area of the carrier is not in between the articles 101, 102. Thus, the length l1₂₂₀ may protrude over an edge of the articles 101, 102, whereby the rest of the carrier 220 can be arranged in between the articles 101, 102. Thus, the length of the part in between the articles would equal (at least to a reasonable degree of accuracy) L3-L1₂₂₀. However, naturally, the whole carrier 220 may be made of non-transparent material. In such a case, the aforementioned length l1₂₂₀ indicates the length of the part of the carrier 220 protruding from an edge of an article of the arrangement, even if the whole carrier 220 is non-transparent. In a preferably embodiment, a length L1 of the first article 101 is at least twice the length L3 of the carrier 220 (i.e. L1 ≥ 2×L3).

A width W3 of the carrier 220 may be e.g. 50 mm - 500 mm, such as 100 mm - 200 mm. The lower limit ensures that a sensor device 211, 212 has sufficient space, and also that a diameter of the aperture 224 can be sufficiently large. The upper limit is beneficial for material costs.

A width (e.g. diameter) w1₂₂₀ of the aperture 224 of the carrier 220 may be e.g. 20 mm - 45 mm, such as 25 mm - 40 mm. It has been found that such a size is sufficient in order for storing the carrier 220 e.g. by suspending from the aperture 224.

## Claims

1. An arrangement (100) comprising
- a first article (101) made of wood based material, the first article (101) having a first length (L1), a first width (W1), and a first thickness (T1), a ratio (L1/T1) of the first length (L1) to the first thickness (T1) being at least ten, and a ratio (W1/T1) of the first width (W1) to the first thickness (T1) being at least five,
- a second article (102) made of wood based material, the second article (102) having a second length (L2), a second width (W2), and a second thickness (T2), a ratio (L2/T2) of the second length (L2) to the second thickness (T2) being at least ten, and a ratio (W2/T2) of the second width (W2) to the second thickness (T2) being at least five, and
- a first sensor device (211) comprising a chip (IC1) and configured to measure a value indicative of a moisture content, wherein
- the first thickness (T1) is unidirectional with the second thickness (T2), and
- at least a part of the chip (IC1) is arranged in between the first article (101) and the second article (102) in the direction of the first thickness (T1); **characterized in that**
- the first sensor device (211) is remotely readable and comprises an antenna (Anti) for transferring data,
- the first article (101) is a wooden veneer (101v) having a thickness from 0.5 mm to 5 mm,
- the second article (102) is a wooden veneer (102v) having a thickness from 0.5 mm to 5 mm, and the arrangement (100) comprises
- a wrapping (300), which surrounds the first article (101), the second article (102), and the first sensor device (211), and comprises plastic.

2. The arrangement (100) of the claim 1, wherein
- the first length (L1) is at least 150 mm, and
- a first distance (d1) in between the chip (IC1) and an edge of the first article (101), wherein the first distance (d1) is unidirectional with the first length (L1), is at least 50 mm.

3. The arrangement (100) of the claim 1 or 2, comprising
- a carrier (220) having a third length (L3) of at least 150 mm in between a first end (E1) of the carrier and a second end (E2) of the carrier and a third thickness (T3) that is less than or equal to the first thickness (T1), wherein
- the carrier (220) comprises a holder (221, 222) that is configured to receive a part of a tool for inserting at least a part of the carrier (220) in between the articles (101, 102),
- the first remotely readable sensor device (211) is fixed to the carrier (220),
- a third distance (d3) between the first end (E1) of the carrier (220) and the chip (IC1) of the first remotely readable sensor device (211) is at least 100 mm, and
- at least a part of the carrier (220) is arranged in between the first article (101) and the second article (102) in the direction of the first thickness (T1); preferably,
- a second remotely readable sensor device (212) configured to measure a value indicative of a moisture content is fixed to the carrier (220) and/or
- the holder (222) is arranged at the second end (E2) of the carrier (220).

4. The arrangement (100) of claim 3, wherein
- the holder (222) is closed towards the second end (E2) of the carrier (220) and open towards the first end (E1) of the carrier (220).

5. The arrangement of the claim 3 or 4, wherein
- the first end (E1) of the carrier is not arranged in between the first article (101) and the second article (102) in the direction of the first thickness (T1); preferably
- the carrier (220) comprises a marking (226) indicative of a preferable location of an end of an article (101, 102), and a distance between the marking (226) and an end of one of the first article (101) is at most 75 mm, and/or
- the first end (E1) of the carrier (220) is not transparent, and/or
- an aperture (224) is arranged at the first end (E1) of the carrier (220), and/or
- the first length (L1) is at least twice the third length (L3).

6. The arrangement (100) of any of the claims 1 to 5, comprising
- a second remotely readable sensor device (212) configured to measure a value indicative of a moisture content, and
- a third remotely readable sensor device (213) configured to measure a value indicative of a moisture content;
preferably,
- each one of the following is at least 50 mm:
• a distance between the chip (IC1) of the first remotely readable sensor device (211) and a chip of the second remotely readable sensor device (212),
• a distance between the chip (IC1) of the first remotely readable sensor device (211) and a chip of the third remotely readable sensor device (213), and
• a distance between a chip of the second remotely readable sensor device (212) and a chip of the third remotely readable sensor device (213).

7. The arrangement (100) of any of the claims 1 to 6, wherein
- the wrapping (300) surrounds the first article (101), the second article (102), and the first remotely readable sensor device (211) such that
- all second distances (d2ᵣ) between the chip (IC1) and the wrapping (300) in all directions (r) are at least 50 mm,
preferably,
- an average moisture content of the first article (101) and the second article (102) is from 1 % to 15 %.

8. The arrangement (100) of claim 7, wherein
- an Sd value of the wrapping (300), as measured according to ISO 12572, is at least 0.5 m; and/or
- the wrapping (300) is waterproof.

9. The arrangement of any of the claims 1 to 8, comprising
- an information carrier (310) comprising information (INFO) indicative of an identity of the first remotely readable sensor device (211);
preferably, when claim 9 depends on claim 6, the information carrier (310) comprises
- information indicative of an identity of the second remotely readable sensor device (212) and information indicative of an identity of the third remotely readable sensor device (213).

10. The arrangement of claim 9, wherein
- the information carrier (310) is arranged in between the first article (101) and the wrapping (300).

11. The arrangement of any of the claims 3 to 10, wherein
- -the first wooden veneer (101v) has a first grain orientation (G1),
- -the second wooden veneer (102v) has a second grain orientation (G2),
- the first grain orientation (G1) is unidirectional with the second grain orientation (G2), and
- a direction of the third length (L3) forms an angle of at most 15 degrees with the first grain orientation (G1) or is unidirectional with the first grain orientation (G1).

12. A method for forming the arrangement (100) of any of the claims 1 to 11, comprising
- receiving
• a first article (101) made of wood based material, the first article (101) having a first length (L1), a first width (W1), and a first thickness (T1), a ratio (L1/T1) of the first length (L1) to the first thickness (T1) being at least ten, and a ratio (W1/T1) of the first width (W1) to the first thickness (T1) being at least five,
• a second article (102) made of wood based material, the second article (102) having a second length (L2), a second width (W2), and a second thickness (T2), a ratio (L2/T2) of the second length (L2) to the second thickness (T2) being at least ten, and a ratio (W2/T2) of the second width (W2) to the second thickness (T2) being at least five, and
• a first sensor device (211) comprising a chip (IC1) configured to provide information indicative of a moisture content, and
- arranging the first and second articles (101, 102) and the first sensor device (211) relative to each other such that the first thickness (T1) is unidirectional with the second thickness (T2), at least a part of the first article (101) overlaps in a direction of the first thickness (T1) with at least a part of the second article (102), and at least a part of the chip (IC1) of the first remotely readable sensor device (211) is in between the first article (101) and the second article (102) in the direction of the first thickness (T1);
**characterized in that**
- the first sensor device (211) is remotely readable and comprises an antenna (Anti) for transferring data,
- the first article (101) is a wooden veneer (101v) having a thickness from 0.5 mm to 5 mm, and
- the second article (102) is a wooden veneer (102v) having a thickness from 0.5 mm to 5 mm; and **in that** the method comprises
- wrapping the first article (101), the second article (102) and the sensor device (211) by a wrapping (300) comprising plastic.

13. The method of claim 12, wherein
- the first remotely readable sensor device (211) is arranged on a carrier (220), wherein
- the carrier (220)
• has a third length (L3) of at least 150 mm in between a first end (E1) of the carrier (220) and a second end (E2) of the carrier (220),
• has a third thickness (T3) that is less than or equal to the first thickness (T1), and
• comprises a holder (221, 222) that is configured to receive a part of a tool (400, 500) for inserting at least a part of the carrier (220) in between the articles (101, 102), the method comprising
- arranging the first article (101) and the second article (102) such that at least a part of the first article (101) overlaps in a direction of the first thickness (T1) with at least a part of the second article (102),
- attaching the tool (400, 500) for inserting at least a part of the carrier (220) in between the articles (101, 102) to the holder (221, 222), and
- by using the tool (400, 500) for inserting at least a part of the carrier (220) in between the articles (101, 102), pushing at least a part of the carrier (220) in between the first article (101) and the second article (102) such that at least a part of the chip (IC1) of the first remotely readable sensor device (211) becomes arranged between the first article (101) and the second article (102) in the direction of the first thickness (T1);
preferably the method further comprises
- pulling the tool (400, 500) for inserting at least a part of the carrier (220) in between the articles (101, 102) from between the articles (101,102) in such a way that at least a part of the chip (IC1) of the first remotely readable sensor device (211) remains between the first article (101) and the second article (102) in the direction of the first thickness (T1).

14. The method of claim 13, wherein
- the carrier (220) comprises a holder (222) that is closed towards the second end (E2) of the carrier (220) and open towards the first end (E1) of the carrier (220), the method comprising
- arranging a part of a rod (400) into the holder (222), and
- by using the rod (400), pushing at least a part of the carrier (220) in between the first article (101) and the second article (102) such that at least a part of the chip (IC1) of the first remotely readable sensor device (211) becomes arranged between the first article (101) and the second article (102) in the direction of the first thickness (T1);
preferably the method further comprises
- pulling the rod (400) from the holder (222) in such a way that at least a part of the chip (IC1) of the first remotely readable sensor device (211) remains between the first article (101) and the second article (102) in the direction of the first thickness (T1).

15. The method of claim 13 or 14, comprising
- pushing at least a part of the carrier (220) in between the first article (101) and the second article (102) such that the first end (E1) of the carrier (220) protrudes from an edge of the first article (101) or the second article (102), whereby
- the carrier (220) is removable from the arrangement (100) by pulling from the first end (E1);
preferably
- the carrier (220) comprises a marking (226) indicative of a preferable location of an end of an article (101, 102), and/or
- the first end (E1) of the carrier (220) is not transparent and/or
- the first end (E1) of the carrier (220) is provided with an aperture (224).

16. The method of any of claims 13 to 15, wherein
- -the first wooden veneer (101v) has a first grain orientation (G1),
- -the second wooden veneer (102v) has a second grain orientation (G2), the method comprising
- arranging the first article (101) and the second article (102) relative to each other in such a way that the first grain orientation (G1) is unidirectional with the second grain orientation (G2), and
- by moving the tool (400, 500) for inserting at least a part of the carrier (220), such as the rod (400), in a direction that forms an angle of at most 15 degrees with the first grain orientation (G1) or is unidirectional with the first grain orientation (G1), pushing at least a part of the carrier (220) in between the first article (101) and the second article (102) such that at least a part of the chip (IC1) becomes arranged between the first article (101) and the second article (102) in the direction of the first thickness (T1).

## Patentansprüche

1. Eine Anordnung (100) die Folgendes umfasst
- einen ersten Gegenstand (101), der aus einem Material auf Holzbasis hergestellt ist, wobei der erste Gegenstand (101) eine erste Länge (L1), eine erste Breite (W1) und eine erste Dicke (T1) aufweist, wobei ein Verhältnis (L1/T1) der ersten Länge (L1) zu der ersten Dicke (T1) mindestens zehn beträgt und ein Verhältnis (W1/T1) der ersten Breite (W1) zu der ersten Dicke (T1) mindestens fünf beträgt,
- einen zweiten Gegenstand (102), der aus einem Material auf Holzbasis hergestellt ist, wobei der zweite Gegenstand (102) eine zweite Länge (L2), eine zweite Breite (W2) und eine zweite Dicke (T2) aufweist, wobei ein Verhältnis (L2/T2) der zweiten Länge (L2) zu der zweiten Dicke (T2) mindestens zehn beträgt und ein Verhältnis (W2/T2) der zweiten Breite (W2) zu der zweiten Dicke (T2) mindestens fünf beträgt, und
- eine erste Sensorvorrichtung (211), die einen Chip (IC1) umfasst und konfiguriert ist, um einen Wert zu messen, der einen Feuchtigkeitsgehalt anzeigt, wobei
- die erste Dicke (T1) unidirektional mit der zweiten Dicke (T2) ist, und
- zumindest ein Teil des Chips (IC1) zwischen dem ersten Artikel (101) und dem zweiten Artikel (102) in Richtung der ersten Dicke (T1) angeordnet ist;
**dadurch gekennzeichnet, dass**
- die erste Sensorvorrichtung (211) fernauslesbar ist und eine Antenne (Anti) zur Übertragung von Daten aufweist,
- der erste Gegenstand (101) ein Holzfurnier (101v) mit einer Dicke von 0,5 mm bis 5 mm ist,
- der zweite Gegenstand (102) ist ein Holzfurnier (102v) mit einer Dicke von 0,5 mm bis 5 mm, und die Anordnung (100) umfasst
- eine Umhüllung (300), die den ersten Gegenstand (101), den zweiten Gegenstand (102) und die erste Sensorvorrichtung (211) umgibt und aus Kunststoff besteht.

2. Die Anordnung (100) nach Anspruch 1, wobei
- die erste Länge (L1) mindestens 150 mm beträgt, und
- ein erster Abstand (d1) zwischen dem Chip (IC1) und einer Kante des ersten Gegenstands (101), wobei der erste Abstand (d1) unidirektional zur ersten Länge (L1) ist, mindestens 50 mm beträgt.

3. Die Anordnung (100) nach Anspruch 1 oder 2, die Folgendes umfasst:
- einen Träger (220) mit einer dritten Länge (L3) von mindestens 150 mm zwischen einem ersten Ende (E1) des Trägers und einem zweiten Ende (E2) des Trägers und einer dritten Dicke (T3), die kleiner als oder gleich der ersten Dicke (T1) ist, wobei
- der Träger (220) umfasst einen Halter (221, 222), der so konfiguriert ist, dass er einen Teil eines Werkzeugs zum Einführen mindestens eines Teils des Trägers (220) zwischen die Gegenstände (101, 102) aufnimmt,
- die erste fernauslesbare Sensorvorrichtung (211) ist an dem Träger (220) befestigt,
- ein dritter Abstand (d3) zwischen dem ersten Ende (E1) des Trägers (220) und dem Chip (IC1) der ersten fernauslesbaren Sensorvorrichtung (211) beträgt mindestens 100 mm, und
- zumindest ein Teil des Trägers (220) zwischen dem ersten Gegenstand (101) und dem zweiten Gegenstand (102) ist in Richtung der ersten Dicke (T1) angeordnet; vorzugsweise,
- eine zweite fernauslesbare Sensorvorrichtung (212), die so konfiguriert ist, dass sie einen Wert misst, der einen Feuchtigkeitsgehalt anzeigt, die am Träger (220) befestigt ist und/oder
- der Halter (222) ist an dem zweiten Ende (E2) des Trägers (220) angeordnet.

4. Die Anordnung (100) nach Anspruch 3, wobei
- der Halter (222) zum zweiten Ende (E2) des Trägers (220) hin geschlossen und zum ersten Ende (E1) des Trägers (220) hin offen ist.

5. Anordnung nach Anspruch 3 oder 4, wobei
- das erste Ende (E1) des Trägers nicht zwischen dem ersten Artikel (101) und dem zweiten Artikel (102) in Richtung der ersten Dicke (T1) angeordnet ist; vorzugsweise
- der Träger (220) eine Markierung (226) aufweist, die eine bevorzugte Lage eines Endes eines Artikels (101, 102) angibt, und ein Abstand zwischen der Markierung (226) und einem Ende eines der ersten Artikel (101) höchstens 75 mm beträgt, und/oder
- das erste Ende (E1) des Trägers (220) nicht transparent ist, und/oder
- eine Öffnung (224) am ersten Ende (E1) des Trägers (220) angeordnet ist, und/oder
- die erste Länge (L1) mindestens das Doppelte der dritten Länge (L3) beträgt.

6. Anordnung (100) nach einem der Ansprüche 1 bis 5, die Folgendes umfasst
- eine zweite fernauslesbare Sensorvorrichtung (212), die so konfiguriert ist, dass sie einen Wert misst, der einen Feuchtigkeitsgehalt angibt, und
- eine dritte fernauslesbare Sensorvorrichtung (213), die so konfiguriert ist, dass sie einen Wert misst, der für einen Feuchtigkeitsgehalt kennzeichnend ist; vorzugsweise,
- jedes der folgenden Elemente beträgt mindestens 50 mm:
• ein Abstand zwischen dem Chip (IC1) der ersten fernauslesbaren Sensorvorrichtung (211) und einem Chip der zweiten fernauslesbaren Sensorvorrichtung (212),
• ein Abstand zwischen dem Chip (IC1) der ersten fernauslesbaren Sensorvorrichtung (211) und einem Chip der dritten fernauslesbaren Sensorvorrichtung (213), und
• ein Abstand zwischen einem Chip der zweiten fernauslesbaren Sensorvorrichtung (212) und einem Chip der dritten fernauslesbaren Sensorvorrichtung (213).

7. Die Anordnung (100) nach einem der Ansprüche 1 bis 6, wobei
- die Umhüllung (300) den ersten Gegenstand (101), den zweiten Gegenstand (102) und die erste fernauslesbare Sensorvorrichtung (211) so umgibt, dass
- alle zweiten Abstände (d2ᵣ) zwischen dem Chip (IC1) und der Umhüllung (300) in allen Richtungen (r) mindestens 50 mm betragen,
vorzugsweise,
- liegt ein durchschnittlicher Feuchtigkeitsgehalt des ersten Gegenstands (101) und des zweiten Gegenstands (102) zwischen 1% und 15%.

8. Die Anordnung (100) nach Anspruch 7, wobei
- ein Sd-Wert der Umhüllung (300), gemessen nach ISO 12572, mindestens 0,5 m beträgt; und/oder
- die Umhüllung (300) wasserdicht ist.

9. Anordnung nach einem der Ansprüche 1 bis 8, die Folgendes umfasst:
- einen Informationsträger (310) mit Informationen (INFO), die eine Identität der ersten fernauslesbaren Sensorvorrichtung (211) anzeigen;
vorzugsweise, wenn Anspruch 9 von Anspruch 6 abhängt, umfasst der Informationsträger (310) Folgendes
- Informationen, die eine Identität der zweiten fernauslesbaren Sensorvorrichtung (212) angeben, und Informationen, die eine Identität der dritten fernauslesbaren Sensorvorrichtung (213) angeben.

10. Die Anordnung nach Anspruch 9, wobei
- der Informationsträger (310) zwischen dem ersten Artikel (101) und der Umhüllung (300) angeordnet ist.

11. Anordnung nach einem der Ansprüche 3 bis 10, wobei
- das erste Holzfurnier (101v) eine erste Faserausrichtung (G1) aufweist,
- das zweite Holzfurnier (102v) eine zweite Faserausrichtung (G2) aufweist,
- die erste Faserausrichtung (G1) unidirektional mit der zweiten Faserausrichtung (G2) ist, und
- eine Richtung der dritten Länge (L3) einen Winkel von höchstens 15 Grad mit der ersten Faserausrichtung (G1) bildet oder unidirektional mit der ersten Faserausrichtung (G1) ist.

12. Verfahren zur Herstellung der Anordnung (100) nach einem der Ansprüche 1 bis 11, das Folgendes umfasst
- Aufnehmen
• eines ersten Gegenstandes (101), der aus einem Material auf Holzbasis hergestellt ist, wobei der erste Gegenstand (101) eine erste Länge (L1), eine erste Breite (W1) und eine erste Dicke (T1) aufweist, wobei ein Verhältnis (L1/T1) der ersten Länge (L1) zu der ersten Dicke (T1) mindestens zehn beträgt und ein Verhältnis (W1/T1) der ersten Breite (W1) zu der ersten Dicke (T1) mindestens fünf beträgt,
• eines zweiten Gegenstandes (102), der aus einem Material auf Holzbasis hergestellt ist, wobei der zweite Gegenstand (102) eine zweite Länge (L2), eine zweite Breite (W2) und eine zweite Dicke (T2) aufweist, wobei ein Verhältnis (L2/T2) der zweiten Länge (L2) zu der zweiten Dicke (T2) mindestens zehn beträgt und ein Verhältnis (W2/T2) der zweiten Breite (W2) zu der zweiten Dicke (T2) mindestens fünf beträgt, und
• einer ersten Sensorvorrichtung (211), die einen Chip (IC1) umfasst, der so konfiguriert ist, dass er Informationen liefert, die einen Feuchtigkeitsgehalt anzeigen, und
- Anordnen des ersten und zweiten Artikels (101, 102) und der ersten Sensorvorrichtung (211) relativ zueinander, so dass die erste Dicke (T1) unidirektional mit der zweiten Dicke (T2) ist, mindestens ein Teil des ersten Artikels (101) in einer Richtung der ersten Dicke (T1) mit mindestens einem Teil des zweiten Artikels (102) überlappt, und mindestens ein Teil des Chips (IC1) der ersten fernauslesbaren Sensorvorrichtung (211) sich zwischen dem ersten Gegenstand (101) und dem zweiten Gegenstand (102) in Richtung der ersten Dicke (T1) befindet;
**dadurch gekennzeichnet, dass**
- die erste Sensorvorrichtung (211) fernauslesbar ist und eine Antenne (Anti) zur Übertragung von Daten aufweist,
- der erste Gegenstand (101) ein Holzfurnier (101v) mit einer Dicke von 0,5 mm bis 5 mm ist, und
- der zweite Gegenstand (102) ein Holzfurnier (102v) mit einer Dicke von 0,5 mm bis 5 mm ist; und dass das Verfahren Folgendes umfasst
- Umhüllen des ersten Gegenstands (101), des zweiten Gegenstands (102) und der Sensorvorrichtung (211) mit einer Umhüllung (300) aus Kunststoff.

13. Verfahren nach Anspruch 12, wobei
- die erste fernauslesbare Sensorvorrichtung (211) auf einem Träger (220) angeordnet ist, wobei
- der Träger (220)
• eine dritte Länge (L3) von mindestens 150 mm zwischen einem ersten Ende (E1) des Trägers (220) und einem zweiten Ende (E2) des Trägers (220) aufweist,
• eine dritte Dicke (T3) aufweist, die kleiner oder gleich der ersten Dicke (T1) ist, und
• einen Halter (221, 222) umfasst, der so konfiguriert ist, dass er einen Teil eines Werkzeugs (400, 500) zum Einführen mindestens eines Teils des Trägers (220) zwischen die Gegenstände (101, 102) aufnimmt, wobei das Verfahren Folgendes umfasst
- Anordnen des ersten Artikels (101) und des zweiten Artikels (102) derart, dass mindestens ein Teil des ersten Artikels (101) in einer Richtung der ersten Dicke (T1) mit mindestens einem Teil des zweiten Artikels (102) überlappt,
- Befestigen des Werkzeugs (400, 500) zum Einführen mindestens eines Teils des Trägers (220) zwischen die Gegenstände (101, 102) an dem Halter (221, 222), und
- unter Verwendung des Werkzeugs (400, 500) Einführen mindestens eines Teils des Trägers (220) zwischen die Gegenstände (101, 102), Schieben mindestens eines Teils des Trägers (220) zwischen den ersten Gegenstand (101) und den zweiten Gegenstand (102), so dass mindestens ein Teil des Chips (IC1) der ersten fernauslesbaren Sensorvorrichtung (211) zwischen dem ersten Gegenstand (101) und dem zweiten Gegenstand (102) in Richtung der ersten Dicke (T1) angeordnet wird;
vorzugsweise umfasst das Verfahren ferner Folgendes
- Ziehen des Werkzeugs (400, 500) zum Einführen mindestens eines Teils des Trägers (220) zwischen die Gegenstände (101, 102) aus dem Zwischenraum zwischen den Gegenständen (101, 102) in der Weise, dass mindestens ein Teil des Chips (IC1) der ersten fernauslesbaren Sensorvorrichtung (211) zwischen dem ersten Gegenstand (101) und dem zweiten Gegenstand (102) in Richtung der ersten Dicke (T1) verbleibt.

14. Verfahren nach Anspruch 13, wobei
- der Träger (220) einen Halter (222) umfasst, der zum zweiten Ende (E2) des Trägers (220) hin geschlossen und zum ersten Ende (E1) des Trägers (220) hin offen ist, wobei das Verfahren Folgendes umfasst
- Anordnen eines Teils eines Stabes (400) in dem Halter (222), und
- unter Verwendung des Stabes (400) zumindest einen Teil des Trägers (220) zwischen den ersten Gegenstand (101) und den zweiten Gegenstand (102) schieben, so dass zumindest ein Teil des Chips (IC1) der ersten fernauslesbaren Sensorvorrichtung (211) zwischen dem ersten Gegenstand (101) und dem zweiten Gegenstand (102) in Richtung der ersten Dicke (T1) angeordnet wird; vorzugsweise umfasst das Verfahren ferner Folgendes
- Ziehen des Stabes (400) aus dem Halter (222) derart, dass zumindest ein Teil des Chips (IC1) der ersten fernauslesbaren Sensorvorrichtung (211) zwischen dem ersten Gegenstand (101) und dem zweiten Gegenstand (102) in Richtung der ersten Dicke (T1) verbleibt.

15. Verfahren nach Anspruch 13 oder 14, das Folgendes umfasst
- Einschieben mindestens eines Teils des Trägers (220) zwischen den ersten Artikel (101) und den zweiten Artikel (102), so dass das erste Ende (E1) des Trägers (220) aus einer Kante des ersten Artikels (101) oder des zweiten Artikels (102) herausragt, wodurch
- der Träger (220) durch Ziehen am ersten Ende (E1) von der Anordnung (100) abnehmbar ist;
vorzugsweise
- weist der Träger (220) eine Markierung (226) auf, die eine bevorzugte Position eines Endes eines Artikels (101, 102) angibt, und/oder
- das erste Ende (E1) des Trägers (220) nicht transparent ist und/oder
- das erste Ende (E1) des Trägers (220) mit einer Öffnung (224) versehen ist.

16. Verfahren nach einem der Ansprüche 13 bis 15, wobei
- das erste Holzfurnier (101v) eine erste Faserausrichtung (G1) aufweist,
- das zweite Holzfurnier (102v) eine zweite Faserausrichtung (G2) aufweist, wobei das Verfahren Folgendes umfasst
- Anordnen des ersten Gegenstands (101) und des zweiten Gegenstands (102) relativ zueinander in einer solchen Weise, dass die erste Faserausrichtung (G1) unidirektional mit der zweiten Faserausrichtung (G2) ist, und
- durch Bewegen des Werkzeugs (400, 500) Einführen mindestens eines Teils des Trägers (220), wie der Stab (400), in einer Richtung, die einen Winkel von höchstens 15 Grad mit der ersten Faserausrichtung (G1) bildet oder unidirektional mit der ersten Faserausrichtung (G1) ist, Schieben mindestens eines Teils des Trägers (220) zwischen den ersten Artikel (101) und den zweiten Artikel (102), so dass mindestens ein Teil des Chips (IC1) zwischen dem ersten Artikel (101) und dem zweiten Artikel (102) in Richtung der ersten Dicke (T1) angeordnet wird.

## Revendications

1. Agencement (100) comprenant
- un premier article (101) fait d'un matériau à base de bois, le premier article (101) ayant une première longueur (L1), une première largeur (W1) et une première épaisseur (T1), un rapport (L1/T1) de la première longueur (L1) à la première épaisseur (T1) étant d'au moins dix, et un rapport (W1/T1) de la première largeur (W1) à la première épaisseur (T1) étant d'au moins cinq,
- un second article (102) fait d'un matériau à base de bois, le second article (102) ayant une deuxième longueur (L2), une seconde largeur (W2) et une deuxième épaisseur (T2), un rapport (L2/T2) de la deuxième longueur (L2) à la deuxième épaisseur (T2) étant d'au moins dix, et un rapport (W2/T2) de la seconde largeur (W2) à la deuxième épaisseur (T2) étant d'au moins cinq, et
- un premier dispositif capteur (211) comprenant une puce (IC1) et configuré pour mesurer une valeur indicative d'une teneur en humidité, dans lequel
- la première épaisseur (T1) est unidirectionnelle avec la deuxième épaisseur (T2), et
- au moins une partie de la puce (IC1) est agencée entre le premier article (101) et le second article (102) dans la direction de la première épaisseur (T1) ; **caractérisé en ce que**
- le premier dispositif capteur (211) est lisible à distance et comprend une antenne (Ant1) pour le transfert de données,
- le premier article (101) est un placage de bois (101v) ayant une épaisseur de 0,5 mm à 5 mm,
- le second article (102) est un placage de bois (102v) ayant une épaisseur de 0,5 mm à 5 mm, et l'agencement (100) comprend
- un emballage (300), qui entoure le premier article (101), le second article (102) et le premier dispositif capteur (211), et comprend du plastique.

2. Agencement (100) selon la revendication 1, dans lequel
- la première longueur (L1) est d'au moins 150 mm, et
- une première distance (d1) entre la puce (IC1) et un bord du premier article (101), dans lequel la première distance (d1) est unidirectionnelle avec la première longueur (L1), est d'au moins 50 mm.

3. Agencement (100) selon la revendication 1 ou 2, comprenant
- un support (220) ayant une troisième longueur (L3) d'au moins 150 mm entre une première extrémité (E1) du support et une seconde extrémité (E2) du support et une troisième épaisseur (T3) qui est inférieure ou égale à la première épaisseur (T1), dans lequel
- le support (220) comprend un dispositif de retenue (221, 222) configuré pour recevoir une partie d'un outil destiné à insérer au moins une partie du support (220) entre les articles (101, 102),
- le premier dispositif capteur lisible à distance (211) est fixé au support (220),
- une troisième distance (d3) entre la première extrémité (E1) du support (220) et la puce (IC1) du premier dispositif capteur lisible à distance (211) est d'au moins 100 mm, et
- au moins une partie du support (220) est agencée entre le premier article (101) et le second article (102) dans la direction de la première épaisseur (T1) ; de préférence,
- un deuxième dispositif capteur lisible à distance (212) configuré pour mesurer une valeur indicative d'une teneur en humidité est fixé au support (220) et/ou
- le dispositif de retenue (222) est agencé à la seconde extrémité (E2) du support (220).

4. Agencement (100) selon la revendication 3, dans lequel
- le dispositif de retenue (222) est fermé vers la seconde extrémité (E2) du support (220) et ouvert vers la première extrémité (E1) du support (220).

5. Agencement selon la revendication 3 ou 4, dans lequel
- la première extrémité (E1) du support n'est pas agencée entre le premier article (101) et le second article (102) dans la direction de la première épaisseur (T1) ; de préférence
- le support (220) comprend un marquage (226) indicatif d'un emplacement préférable d'une extrémité d'un article (101, 102), et une distance entre le marquage (226) et une extrémité de l'un du premier article (101) est au plus de 75 mm, et/ou
- la première extrémité (E1) du support (220) n'est pas transparente, et/ou
- une ouverture (224) est agencée à la première extrémité (E1) du support (220), et/ou
- la première longueur (L1) est au moins le double de la troisième longueur (L3).

6. Agencement (100) selon l'une quelconque des revendications 1 à 5, comprenant
- un deuxième dispositif capteur lisible à distance (212) configuré pour mesurer une valeur indicative d'une teneur en humidité, et
- un troisième dispositif capteur lisible à distance (213) configuré pour mesurer une valeur indicative d'une teneur en humidité ;
de préférence,
- chacun des éléments suivants est d'au moins 50 mm :
• une distance entre la puce (IC1) du premier dispositif capteur lisible à distance (211) et une puce du deuxième dispositif capteur lisible à distance (212),
• une distance entre la puce (IC1) du premier dispositif capteur lisible à distance (211) et une puce du troisième dispositif capteur lisible à distance (213), et
• une distance entre une puce du deuxième dispositif capteur lisible à distance (212) et une puce du troisième dispositif capteur lisible à distance (213).

7. Agencement (100) selon l'une quelconque des revendications 1 à 6, dans lequel
- l'emballage (300) entoure le premier article (101), le second article (102) et le premier dispositif capteur lisible à distance (211) de sorte que
- toutes les deuxièmes distances (d2ᵣ) entre la puce (IC1) et l'emballage (300) dans toutes les directions (r) sont d'au moins 50 mm,
de préférence,
- une teneur moyenne en humidité du premier article (101) et du second article (102) est de 1 % à 15 %.

8. Agencement (100) selon la revendication 7, dans lequel
- une valeur Sd de l'emballage (300), telle que mesurée selon la norme ISO 12572, est d'au moins 0,5 m ; et/ou
- l'emballage (300) est étanche.

9. Agencement selon l'une quelconque des revendications 1 à 8, comprenant
- un support d'informations (310) comprenant des informations (INFO) indicatives d'une identité du premier dispositif capteur lisible à distance (211) ;
de préférence, lorsque la revendication 9 dépend de la revendication 6, le support d'informations (310) comprend
- des informations indicatives d'une identité du deuxième dispositif capteur lisible à distance (212) et des informations indicatives d'une identité du troisième dispositif capteur lisible à distance (213).

10. Agencement selon la revendication 9, dans lequel
- le support d'informations (310) est agencé entre le premier article (101) et l'emballage (300).

11. Agencement selon l'une quelconque des revendications 3 à 10, dans lequel
- le premier placage de bois (101v) a une première orientation des grains (G1), le second placage de bois (102v) a une seconde orientation des grains (G2),
- la première orientation des grains (G1) est unidirectionnelle avec la seconde orientation des grains (G2), et
- une direction de la troisième longueur (L3) forme un angle d'au plus 15 degrés avec la première orientation des grains (G1) ou est unidirectionnelle avec la première orientation des grains (G1).

12. Procédé de formation de l'agencement (100) selon l'une quelconque des revendications 1 à 11, comprenant
- la réception
• d'un premier article (101) fait d'un matériau à base de bois, le premier article (101) ayant une première longueur (L1), une première largeur (W1) et une première épaisseur (T1), un rapport (L1/T1) de la première longueur (L1) à la première épaisseur (T1) étant d'au moins dix, et un rapport (W1/T1) de la première largeur (W1) à la première épaisseur (T1) étant d'au moins cinq,
• d'un second article (102) fait d'un matériau à base de bois, le second article (102) ayant une deuxième longueur (L2), une seconde largeur (W2) et une deuxième épaisseur (T2), un rapport (L2/T2) de la deuxième longueur (L2) à la deuxième épaisseur (T2) étant d'au moins dix, et un rapport (W2/T2) de la seconde largeur (W2) à la deuxième épaisseur (T2) étant d'au moins cinq, et
• d'un premier dispositif capteur (211) comprenant une puce (IC1) configurée pour fournir des informations indicatives d'une teneur en humidité, et
- l'agencement des premier et second articles (101, 102) et du premier dispositif capteur (211) les uns par rapport aux autres de sorte que la première épaisseur (T1) soit unidirectionnelle avec la deuxième épaisseur (T2), au moins une partie du premier article (101) chevauche dans une direction de la première épaisseur (T1) au moins une partie du second article (102), et au moins une partie de la puce (IC1) du premier dispositif capteur lisible à distance (211) est entre le premier article (101) et le second article (102) dans la direction de la première épaisseur (T1) ;
**caractérisé en ce que**
- le premier dispositif capteur (211) est lisible à distance et comprend une antenne (Ant1) pour le transfert de données,
- le premier article (101) est un placage de bois (101v) ayant une épaisseur de 0,5 mm à 5 mm, et
- le second article (102) est un placage de bois (102v) ayant une épaisseur de 0,5 mm à 5 mm ; et **en ce que** le procédé comprend
- l'emballage du premier article (101), du second article (102) et du dispositif capteur (211) par un emballage (300) comprenant du plastique.

13. Procédé selon la revendication 12, dans lequel
- le premier dispositif capteur lisible à distance (211) est agencé sur un support (220), dans lequel
- le support (220)
• a une troisième longueur (L3) d'au moins 150 mm entre une première extrémité (E1) du support (220) et une seconde extrémité (E2) du support (220),
• a une troisième épaisseur (T3) qui est inférieure ou égale à la première épaisseur (T1), et
• comprend un dispositif de retenue (221, 222) qui est configuré pour recevoir une partie d'un outil (400, 500) destiné à insérer au moins une partie du support (220) entre les articles (101, 102), le procédé comprenant
- l'agencement du premier article (101) et du second article (102) de sorte qu'au moins une partie du premier article (101) chevauche dans une direction de la première épaisseur (T1) au moins une partie du second article (102),
- la fixation de l'outil (400, 500) destiné à insérer au moins une partie du support (220) entre les articles (101, 102) au dispositif de retenue (221, 222), et
- à l'aide de l'outil (400, 500) destiné à insérer au moins une partie du support (220) entre les articles (101, 102), la poussée d'au moins une partie du support (220) entre le premier article (101) et le second article (102) de sorte qu'au moins une partie de la puce (IC1) du premier dispositif capteur lisible à distance (211) soit agencée entre le premier article (101) et le second article (102) dans la direction de la première épaisseur (T1) ;
de préférence, le procédé comprend en outre
- la traction de l'outil (400, 500) destiné à insérer au moins une partie du support (220) entre les articles (101, 102) depuis entre les articles (101, 102) de telle sorte qu'au moins une partie de la puce (IC1) du premier dispositif capteur lisible à distance (211) reste entre le premier article (101) et le second article (102) dans la direction de la première épaisseur (T1).

14. Procédé selon la revendication 13, dans lequel
- le support (220) comprend un dispositif de retenue (222) qui est fermé vers la seconde extrémité (E2) du support (220) et ouvert vers la première extrémité (E1) du support (220), le procédé comprenant
- l'agencement d'une partie d'une tige (400) dans le dispositif de retenue (222), et
- à l'aide de la tige (400), la poussée d'au moins une partie du support (220) entre le premier article (101) et le second article (102) de sorte qu'au moins une partie de la puce (IC1) du premier dispositif capteur lisible à distance (211) devient agencée entre le premier article (101) et le second article (102) dans la direction de la première épaisseur (T1) ;
de préférence, le procédé comprend en outre
- la traction de la tige (400) du support (222) de telle sorte qu'au moins une partie de la puce (IC1) du premier dispositif capteur lisible à distance (211) reste entre le premier article (101) et le second article (102) dans la direction de la première épaisseur (T1).

15. Procédé selon la revendication 13 ou 14, comprenant
- la traction d'au moins une partie du support (220) entre le premier article (101) et le second article (102) de sorte que la première extrémité (E1) du support (220) dépasse d'un bord du premier article (101) ou du second article (102), moyennant quoi
- le support (220) peut être retiré de l'agencement (100) par traction depuis la première extrémité (E1) ;
de préférence
- le support (220) comprend un marquage (226) indicatif d'un emplacement préférable d'une extrémité d'un article (101, 102), et/ou
- la première extrémité (E1) du support (220) n'est pas transparente et/ou
- la première extrémité (E1) du support (220) est pourvue d'une ouverture (224).

16. Procédé selon l'une quelconque des revendications 13 à 15, dans lequel
- le premier placage de bois (101v) a une première orientation des grains (G1),
- le second placage de bois (102v) a une seconde orientation des grains (G2), le procédé comprenant
- l'agencement du premier article (101) et du second article (102) l'un par rapport à l'autre de telle sorte que la première orientation des grains (G1) est unidirectionnelle avec la seconde orientation des grains (G2), et
- par déplacement de l'outil (400, 500) destiné à insérer au moins une partie du support (220), telle que la tige (400), dans une direction qui forme un angle d'au plus 15 degrés avec la première orientation des grains (G1) ou est unidirectionnelle avec la première orientation des grains (G1), la poussée d'au moins une partie du support (220) entre le premier article (101) et le second article (102) de sorte qu'au moins une partie de la puce (IC1) devient agencée entre le premier article (101) et le second article (102) dans la direction de la première épaisseur (T1).
